# EUROPÄISCHE PATENTSCHRIFT

(11) **EP 4 366 810 B1**
(45) Veröffentlichungstag und Bekanntmachung des Hinweises auf die Patenterteilung: **19.03.2025**
(21) Anmeldenummer: 22744421.3
(22) Anmeldetag: 01.07.2022
(51) Int. Cl.: A61M 16/08, A61M 16/20

(54) **EXSPIRATIONSVENTIL-BAUGRUPPE UND ATEMGAS-LEITUNGSBAUGRUPPE MIT EINER SOLCHEN**
EXPIRATION VALVE ASSEMBLY AND BREATHING GAS LINE ASSEMBLY COMPRISING SUCH AN ASSEMBLY
ENSEMBLE SOUPAPE D'EXPIRATION ET ENSEMBLE CONDUITE DE GAZ RESPIRATOIRE ÉQUIPÉ D'UN TEL ENSEMBLE

(30) Priorität: 06.07.2021 DE 102021117375
(43) Veröffentlichungstag der Anmeldung: 15.05.2024
(73) Patentinhaber: Hamilton Medical AG, 7402 Bonaduz (CH)
(72) Erfinder: LIPINSKI, Kerstin, 7014 Trin (CH); HALTINER, Kim, 7000 Chur (CH)
(74) Vertreter: Ruttensperger Lachnit Trossin Gomoll
(86) Internationale Anmeldenummer: PCT/EP2022/068277
(87) Internationale Veröffentlichungsnummer: WO 2023/280710

(56) Entgegenhaltungen:
- EP-A1- 3 360 595
- WO-A1-2018/077618
- WO-A1-2018/210958
- DE-A1- 102019 121 712
- US-A1- 2020 179 640

## Beschreibung

Die vorliegende Erfindung betrifft eine Exspirationsventil-Baugruppe für eine Beatmungsvorrichtung zur künstlichen Beatmung eines Patienten, umfassend
- einen Exspirationskanal, welcher an einem Ende einen Exspirationseinlass zum Einleiten einer exspiratorischen Atemgasströmung in den Exspirationskanal sowie eine Einlass-Anschlussformation aufweist, die zum Anschluss an eine zum Patienten führende Exspirations-Atemgasleitung ausgebildet ist, und welcher an seinem anderen Ende einen Exspirationsauslass zum Abblasen von exspiratorischem Atemgas aufweist, wobei der Exspirationskanal ein Exspirationsventil aufweist, welches durch eine exspiratorische Atemgasströmung in Exspirationsrichtung vom Exspirationseinlass zum Exspirationsauslass in eine die exspiratorische Atemgasströmung durchlassende Durchlassstellung bewegbar ist,
- einen Inspirationskanal, welcher an einem Ende einen Inspirationseinlass zum Einleiten einer inspiratorischen Atemgasströmung in den Inspirationskanal sowie eine Einlass-Verbindungsformation aufweist, die zur Verbindung mit einer inspiratorisches Atemgas liefernden Atemgasquelle ausgebildet ist, und welcher an seinem anderen Ende einen Inspirationsauslass zum Auslassen von inspiratorischem Atemgas sowie eine Auslass-Verbindungsformation aufweist, die zur Verbindung mit einer zum Patienten führenden Inspirations-Atemgasleitung ausgebildet ist,
- einen Steuerkanal, welcher an einem Abzweigort von dem Inspirationskanal abzweigt und derart zu dem Exspirationsventil führt, dass das Exspirationsventil durch inspiratorisches Atemgas in eine die exspiratorische Atemgasströmung sperrende Sperrstellung belastbar ist.

Eine solche Exspirationsventil-Baugruppe, welche dazu ausgebildet ist, sowohl eine exspiratorische wie auch eine inspiratorische Atemgasströmung zu leiten, ist beispielsweise aus der WO 2021/018902 A1 bekannt.

Die aus der WO 2021/018902 A1 bekannte Exspirationsventil-Baugruppe ist Teil einer Atemgas-Leitungsbaugruppe, mit einem Schlauch als einem an die Exspirationsventil-Baugruppe angeschlossenen Leitungsbauteil. Dieser Schlauch ist als Bi-Lumen-Schlauch derart ausgebildet, dass er innerhalb seiner äußersten Schlauchhülle sowohl die Exspirations-Atemgasleitung als auch die Inspirations-Atemgasleitung aufweist. An seinem distalen, also im Betrieb vom Patienten fernliegenden, Längsende ist der bekannte Bi-Lumen-Schlauch mit der Exspirationsventil-Baugruppe verbunden. An seinem proximalen, also im Betrieb dem Patienten näher gelegenen, Längsende ist an den Schlauch ein Y-Verbindungselement mit einer Rückschlag-Ventilbaugruppe angeordnet. Das Y-Verbindungselement führt zwischen seinem distalen Längsende mit zwei Schenkeln und seinem proximalen Längsende mit einem Schenkel die distal angeschlossenen Atemgasleitungen: Exspirations- und Inspirations-Atemgasleitung in eine gemeinsame bidirektionale Atemgasleitung zusammen.

Die Rückschlag-Ventilbaugruppe dient dazu, im distal der Rückschlag-Ventilbaugruppe gelegenen Bereich der jeweils gerade inaktiven Atemgasleitung aus Inspirations- und Exspirations-Atemgasleitung vorhandene Atemgassäulen von der jeweils anderen, aktiven Atemgasleitung abzukoppeln. Dadurch soll Atemgas nur durch die in der jeweiligen Beatmungsphase gewünschte, aktive Atemgasleitung strömen.

Jedoch fordert das Y-Verbindungselement durch die Aufnahme der Rückschlag-Ventilbaugruppe verhältnismäßig viel Bauraum und wird sperrig. Dies ist gerade für das stets patientennah angeordnete Y-Verbindungselement nachteilig.

Das im Exspirationskanal angeordnete bekannte Exspirationsventil sorgt für eine Sperrung des Exspirationskanals, indem das Exspirationsventil dann, wenn während einer Inspirationsphase inspiratorisches Atemgas in Inspirationsrichtung durch den Inspirationskanal strömt, von abgezweigtem inspiratorischem Atemgas in die Sperrstellung belastet wird. Bei spontaner Inspiration des Patienten sorgt der vom Patienten selbst während der spontanen Inspiration erzeugte Unterdruck für eine Belastung des Exspirationsventils in die Sperrstellung. In jedem der beiden genannten Fälle besteht während einer Inspirationsphase am Exspirationsventil zwischen seiner in Exspirationsrichtung stromaufwärtigen Seite und seiner stromabwärtigen Seite ein Druckgefälle, welches das Exspirationsventil in die Sperrstellung belastet.

In der aus der WO 2021/018902 A1 bekannten Atemgas-Leitungsbaugruppe sorgt ein Rückschlagventil im Y-Verbindungselement für eine ähnliche Sperrung der Inspirations-Atemgasleitung während einer Exspiration. Es verhindert eine Atemgasströmung in der Inspirations-Atemgasleitung in der der Inspirationsrichtung entgegengesetzten Exspirationsrichtung.

Als weiterer Hintergrund des Stands der Technik sei ergänzend auf die EP 2 663 354 B1 verwiesen.

Aufgabe der vorliegenden Erfindung ist es, eine technische Lehre anzugeben, welche es ermöglicht, die in der jeweiligen Beatmungsphase aus Inspiration und Exspiration vorübergehend inaktive Atemgasleitung für eine Durchströmung in der für die jeweils inaktive Atemgasleitung falsche Richtung zu sperren, ohne nahe am Patienten sperrige medizintechnische Komponenten dauerhaft anordnen zu müssen.

Die vorliegende Erfindung löst diese Aufgabe dadurch, dass im Inspirationskanal einer Exspirationsventil-Baugruppe der eingangs genannten Art ein Einwegventil angeordnet ist, welches eine inspiratorische Atemgasströmung in Inspirationsrichtung vom Inspirationseinlass zum Inspirationsauslass zulässt und eine Atemgasströmung in entgegengesetzter Richtung unterbindet.

In vorteilhafter Weise sorgt so die stets fern vom Patienten auf der distalen Seite eines Beatmungsschlauchs oder dergleichen angeordnete Exspirationsventil-Baugruppe während einer Inspiration für eine Sperrung der Exspirations-Atemgasleitung und während einer Exspiration für eine Sperrung der Inspirations-Atemgasleitung. Das hierfür notwendige Einwegventil kann also zum einen in einer verhältnismäßig kompakten Baugruppe und zum anderen mit Entfernung vom beatmeten Patienten angeordnet werden. Selbst wenn die Exspirationsventil-Baugruppe durch die Anordnung des Einwegventils darin gegenüber der aus dem Stand der Technik bekannten Exspirationsventil-Baugruppe an Bauvolumen zunehmen würde, geschähe dies in so großer Entfernung vom beatmeten Patienten, dass dieser davon nicht eingeschränkt würde.

Das Einwegventil ist bevorzugt ein einfaches Rückschlagventil mit einem Ventilsitz und einem Ventilkörper, welcher in seiner die Inspirations-Atemgasleitung verschließenden Schließstellung körperlich am Ventilsitz anliegt, und zur Durchströmung der Inspirations-Atemgasleitung vom Ventilsitz entfernbar ist. Um sicherzustellen, dass die Inspirations-Atemgasleitung nur in Inspirationsrichtung durchströmbar ist, ist der Ventilkörper an seinem Anbringungsort in der Exspirationsventil-Baugruppe nur längs der lokalen Inspirationsrichtung vom Ventilsitz abhebbar. Dadurch kann das Einwegventil durch die inspiratorische Atemgasströmung selbst in seine vom Ventilsitz abgehobene und folglich eine Durchströmung gestattende Offenstellung verstellt werden, während eine Atemgasströmung in entgegengesetzter Richtung den Ventilkörper zurück auf seinen Ventilsitz und damit in die Schließstellung belastet.

Der Ventilkörper kann durch eine elastische Kraft, beispielsweise durch ein Vorspannmittel, wie etwa eine Vorspannfeder oder ein elastisches Polymer-Vorspannelement, in die Schließstellung vorgespannt sein. Eine vorteilhafte Vereinfachung durch möglichst geringe Bauteileanzahl kann dadurch erhalten werden, dass der Ventilkörper sein eigenes Vorspannelement ist. Bevorzugt ist daher der Ventilkörper durch die inspiratorische Atemgasströmung zwischen einer Schließgestalt und einer Öffnungsgestalt verformbar. In der Schließgestalt liegt der Ventilkörper am Ventilsitz an und verschließt den Inspirationskanal. In der Öffnungsgestalt ist der Ventilkörper wenigstens abschnittsweise vom Ventilsitz abgehoben und gestattet eine Durchströmung des Inspirationskanals in Inspirationsrichtung. Ein zusätzliches Vorspannbauteil kann dadurch entfallen.

Gemäß der vorliegenden Erfindung kann der Ventilkörper als schwenkbare Klappe ausgebildet sein, wobei zur Realisierung der oben genannten Verformbarkeit zwischen Schließgestalt und Öffnungsgestalt ein Scharnier, welches die Schwenkbewegung des klappenartig ausgebildeten Ventilkörpers gestattet, bevorzugt als Filmscharnier ausgebildet ist.

Alternativ, und wegen der Möglichkeit einer symmetrischen Durchströmung bevorzugt, kann der Ventilkörper als Scheibe ausgebildet sein, welche in einem von ihrem Scheibenrand entfernt gelegenen zentralen Scheibenbereich gehaltert ist, sodass sich der Scheibenrand relativ zum gehalterten zentralen Scheibenbereich durch Verformung der Scheibe quer zur Scheibenfläche bewegen kann. Der Ventilsitz kann dann durch eine Ringfläche gebildet sein, auf welcher ein radial außerhalb des zentralen Scheibenbereichs gelegener Scheibenrandbereich in der Schließstellung des Einwegventils aufliegt, und von welcher der Scheibenrandbereich durch Verformung des scheibenförmigen Ventilkörpers in die Öffnungsgestalt abhebbar ist.

Zur Halterung des Ventilkörpers kann dieser im zentralen Scheibenbereich eine ihn durchsetzende Öffnung aufweisen, welche von einem Halterungsbauteil durchsetzt ist. Das Halterungsbauteil weist bevorzugt beiderseits der durchsetzten Öffnung einen größeren Querschnitt auf als die durchsetzte Öffnung, sodass der Ventilkörper sehr effektiv formschlüssig durch das Halterungsbauteil gehaltert sein kann.

Bevorzugt sind der Ventilsitz und das Halterungsbauteil des Ventilkörpers als ein einstückiges Bauteil ausgebildet. Das Einwegventil ist bevorzugt als vormontierte Baugruppe, umfassend das Basisbauteil mit dem Ventilsitz und dem Halterungsbauteil, in den Inspirationskanal einführbar. Bevorzugt ist das Bauteil, besonders bevorzugt als einstückiges Bauteil, mit Ventilsitz und Halterungsbauteil nach Einführung in den Inspirationskanal in diesem festlegbar, entweder durch Kleben, Schweißen, wie beispielsweise Ultraschall- oder Reibschweißen, oder einfach reibschlüssig durch Reibung zwischen einer der Innenoberfläche der Inspirationskanalwand zugewandten Außenoberfläche einer Bauteilwand und der Innenoberfläche. Zur erleichterten Einführung kann das Halterungsbauteil zumindest mit seinem am Inspirationskanal festlegbaren Festlegeabschnitt rotationssymmetrisch oder mit sich längs der Umfangsrichtung wiederholenden Gestaltabschnitten ausgebildet sein, wenn der Abschnitt des Inspirationskanals, in welchen das Basisbauteil mit dem Einwegventil eingeführt wird, ebenfalls rotationssymmetrisch ausgebildet ist. Dann kommt es auf eine Orientierung des Basisbauteils bezüglich der Einführachse bei der Montage vorteilhaft nicht an.

Zur Erleichterung eines thermischen Festlegens durch Aufschmelzen einander gegenüberliegender Grenzflächen, wie etwa beim Schweißen, sind wenigstens der Festlegeabschnitt des Basisbauteils und der ihn aufnehmende Abschnitt des Inspirationskanals zumindest an den einander gegenüberliegenden und einander berührenden Flächen aus kompatiblen thermoplastischen Kunststoffen hergestellt. Vorzugsweise ist zur Vereinfachung der Fertigung das gesamte Basisbauteil mit Ventilsitz und Halterungsbauteil oder/und der gesamte Inspirationskanal durch kompatible thermoplastische Werkstoffe gebildet.

Der Ventilkörper, insbesondere der scheibenförmige Ventilkörper, kann aus einem thermoplastischen Elastomer oder allgemein aus einem Elastomer gebildet sein, wie etwa aus Silikon. Der Werkstoff des Ventilkörpers muss nicht mit dem Werkstoff des Ventilsitzes oder/und des Halterungsbauteils oder/und mit dem die Inspirationskanalwand bildenden Werkstoff kompatibel sein. Vorzugsweise ist er dies auch nicht, um unerwünschte Zufallsverbindungen zwischen dem Ventilkörper und dem Ventilsitz oder/und der Inspirationskanalwand zu vermeiden.

Die Inspirationsrichtung und die Exspirationsrichtung sind funktionell entgegengesetzte Richtungen, was jedoch nicht bedeutet, dass die inspiratorische Atemgasströmung und die exspiratorische Atemgasströmung an jedem Ort in der Exspirationsventil-Baugruppe in jeweils entgegengesetzte Strömungsrichtungen strömen. Der Exspirationskanal und der Inspirationskanal können abschnittsweise parallel verlaufen, verlaufen jedoch üblicherweise nicht längs ihrer gesamten gemeinsamen Erstreckung parallel, da beispielsweise der bevorzugt im Anordnungsbereich des Exspirationsventils gelegene Exspirationsauslass in die Außenumgebung der Exspirationsventil-Baugruppe öffnet, während der Inspirationseinlass an eine Atemgasquelle angeschlossen ist, wie beispielsweise an einen unter Druck stehenden Atemgasvorrat oder/und an ein Gebläse oder dergleichen. Soweit sich der Exspirationskanal und der Inspirationskanal lokal parallel an oder in der Exspirationsventil-Baugruppe erstrecken strömen in diesem parallelen Bereich die exspiratorische und die inspiratorische Atemgasströmung in der Regel tatsächlich in entgegengesetzte Strömungsrichtungen.

Die Einlass-Anschlussformation, die Einlass-Verbindungformation und die Auslass-Verbindungsformation können beliebige Gestalten aufweisen, welche einen Anschluss einer weiteren Leitung oder eines weiteren Kanals ermöglichen. Die genannten Formationen können jeweils ein Teil einer pneumatischen Schnellkupplung sein oder können einfach nur durch entweder einen Kanalstutzen oder eine Kanalbuchse gebildet sein. Fachleute wissen in der Regel, wie Atemgasleitungen an eine Einlass-Anschlussformation und eine Auslass-Verbindungsformation anzuschließen sind. Ebenso ist Fachleuten bekannt, wie eine Einlass-Verbindungsformation mit einem Atemgasvorrat, also etwa einem Behälter mit darin gespeichertem unter Druck stehendem Atemgas oder einem Beatmungsgerät mit einem Gebläse, strömungstechnisch wirksam verbunden werden kann.

Um sicherzugehen, dass das Exspirationsventil unabhängig von der Funktionsfähigkeit des Einwegventils durch inspiratorisches Atemgas in die Sperrstellung belastet werden kann, ist bevorzugt der Abzweigort in Inspirationsrichtung stromaufwärts des Einwegventils gelegen.

Das Exspirationsventil ist bevorzugt als ein an sich bekanntes Membranventil ausgebildet, mit einer orthogonal zu ihrer Haupterstreckungsfläche auslenkbaren Membran als einem Ventilkörper und mit einem stirnseitigen Längsende eines Abschnitts des Exspirationskanals als einem ringförmigen Ventilsitz, auf welchem der Ventilkörper in der Sperrstellung aufliegt.

Bevorzugt weist das Exspirationsventil in an sich bekannter Weise einen radial inneren, den Exspirationseinlass aufweisenden einlassseitigen Exspirationskanalabschnitt mit Ventilsitz und einen radial außerhalb des einlassseitigen Exspirationskanalabschnitts gelegenen, den Exspirationsauslass aufweisenden auslassseitigen Exspirationskanalabschnitt auf. Nur in der Durchlassstellung, also mit vom Ventilsitz abgehobenem Ventilkörper, kann exspiratorisches Atemgas vom einlassseitigen in den auslassseitigen Exspirationskanalabschnitt überströmen. Aufgrund der verhältnismäßig großen Membranfläche kann auf der vom Ventilsitz weg weisenden Seite des bevorzugten Membranventilkörpers eine Kammer ausgebildet sein, welche mit dem Steuerkanal kommuniziert, sodass durch den Steuerkanal inspiratorisches Atemgas in die Kammer strömen und dort den Membranventilkörper zum Ventilsitz hin, also in seine Sperrstellung, belasten kann.

Der Membranventilkörper hat bevorzugt eine inspiratorische Membranfläche, welche der Kammer zugewandt ist und welche von inspiratorischem Atemgas benetzbar ist. Auf der entgegengesetzten Seite, welche dem Exspirationseinlass zugewandt ist, weist der Membranventilkörper bevorzugt eine exspiratorische Membranfläche auf, welche von exspiratorischem Atemgas aus dem einlassseitigen Exspirationskanalabschnitt anströmbar ist. Die exspiratorische Membranfläche ist bei Betrachtung des Exspirationsventils im geschlossenen Zustand als einem Bezugszustand innerhalb des Ventilsitzes gelegen. Bevorzugt ist die von inspiratorischem Atemgas benetzbare inspiratorische Membranfläche Fläche des Membranventilkörpers größer als die von exspiratorischem Atemgas anströmbare exspiratorische Membranfläche, um während eines Inspirationsvorgangs das Exspirationsventil durch inspiratorisches Atemgas sicher geschlossen halten zu können. Ein vorteilhaftes Flächenverhältnis von inspiratorischer zu exspiratorischer Membranfläche liegt im Bereich von 1,5 bis 2. Stärker bevorzugt liegt das Flächenverhältnis im Bereich von 1,7 bis 1,9. Besonders bevorzugt beträgt das Flächenverhältnis 1,8.

Grundsätzlich können der Inspirationskanal und der Exspirationskanal beliebige Verläufe zwischen ihrem jeweiligen Einlass und ihrem jeweiligen Auslass aufweisen. Zur Vermeidung unnötiger Verwirbelungen und Strömungswiderstände ist es jedoch bevorzugt, dass ein dem Inspirationseinlass näher als dem Inspirationsauslass gelegener Inspirations-Einlassabschnitt des Inspirationskanals längs einer Inspirations-Einlassachse verläuft. Bevorzugt weist der Inspirations-Einlassabschnitt den Inspirationseinlass auf und erstreckt sich ausgehend von diesem.

Zur Klarstellung ist angemerkt, dass in der vorliegenden Anmeldung mit dem Begriff "Achse" eine geradlinige Bahn bezeichnet ist.

In der WO 2021/018902 A1 verläuft die Ventilbewegungsbahn, längs welcher der Ventilkörper des Exspirationsventils von seinem Ventilsitz abhebbar ist, orthogonal zur Inspirations-Einlassachse. Die bekannte Ventilbewegungsbahn verläuft außerdem parallel zu einem Inspirations-Auslassabschnitt, welcher dem Inspirationsauslass näher gelegen ist als dem Inspirationseinlass. Der bekannte Inspirations-Auslassabschnitt verläuft längs einer Inspirations-Auslassachse. Außerdem verläuft die bekannte Ventilbewegungsbahn parallel zu einem längs einer Exspirations-Einlassachse verlaufenden Exspirations-Einlassabschnitt, welcher dem Exspirationseinlass näher gelegen ist als dem Exspirationsauslass. Bei dieser Kinematik des Ventilkörpers kann in manchen Betriebssituationen die Schwerkraft eine unvorteilhafte Wirkung auf das Exspirationsventil oder zumindest keine vorteilhafte Wirkung auf dieses haben.

Grundsätzlich ist der Ventilkörper des Exspirationsventils in die Sperrstellung vorgespannt, in der Regel durch die Materialelastizität des bevorzugten Membranventilkörpers. Um in den meisten Betriebssituationen zusätzlich dafür sorgen zu können, dass die Schwerkraft das Exspirationsventil in die Sperrstellung belastet und somit die Funktionssicherheit des Exspirationsventils erhöht, ist an der vorliegend diskutierten Exspirationsventil-Baugruppe bevorzugt vorgesehen, dass eine Ventilbewegungsbahn, längs welcher ein Ventilkörper, insbesondere der zuvor erwähnte Membranventilkörper, des Exspirationsventils von einem Ventilsitz, insbesondere dem zuvor genannten Ventilsitz, des Exspirationsventils aus seiner Sperrstellung abhebbar und an den Ventilsitz annäherbar ist, relativ zur Inspirations-Einlassachse um einen Anstellwinkel im Bereich von 10° bis 45°, vorzugsweise im Bereich von 15° bis 35°, geneigt ist. Bevorzugt kommt die hier diskutierte Exspirationsventil-Baugruppe an einem Notfall-Beatmungsgerät zum Einsatz, welches in Kranken- oder Unfall-Nottransportmittel, wie Rettungswagen, Rettungshubschrauber usw., mitgeführt wird. Dementsprechend unvorhersehbar ist die Orientierung der Exspirationsventil-Baugruppe im Notfalleinsatz. Die angegebene Anstellung des Exspirationsventils bezüglich der Ventilbewegungsbahn hat gezeigt, dass in den meisten Betriebssituationen die Exspirationsventil-Baugruppe derart orientiert zum Einsatz kommt, dass wenigstens ein Anteil der auf den Ventilkörper wirkenden Schwerkraft diesen in die Sperrstellung belastet.

Eine in der Anwendungssituation mit an den Anschluss- und Verbindungsformationen der Exspirationsventil-Baugruppe angeschlossenen weiteren Leitungen vorteilhaft kompakte Exspirationsventil-Baugruppe kann dadurch erhalten werden, dass ein dem Exspirationseinlass näher als dem Exspirationsauslass gelegener Exspirations-Einlassabschnitt des Exspirationskanals längs einer Exspirations-Einlassachse verläuft. Bevorzugt ist die Ventilbewegungsbahn um eine Parallele zur Exspirations-Einlassachse bezüglich der Inspirations-Einlassachse geneigt.

Bevorzugt sind die Exspirations-Einlassachse und die Inspirations-Einlassachse orthogonal zueinander orientiert. Die beiden als den jeweiligen Kanalabschnitt zentral durchsetzend gedachten Einlassachsen müssen sich dabei nicht schneiden, sondern können in einem Abstand aneinander vorbeigehen.

Bevorzugt ist der Ventilsitz eben bzw. hat eine in einer Ebene gelegene Ventilsitzfläche. Falls die Ventilsitzfläche eine Erstreckung längs der Ventilbewegungsbahn aufweist, etwa weil sie konisch oder negativ-konisch ist, ist die oben genannte Ebene der Ventilsitzfläche nicht als mathematische Ebene zu verstehen, sondern als technische Ebene mit einer geringen Erstreckung längs der Ventilbewegungsbahn.

Dann, wenn die Ventilbewegungsbahn zur Inspirations-Einlassachse gemäß obiger Erläuterung geneigt ist, ist bevorzugt auch der Ventilsitz bzw. die Erstreckungsebene der ebenen Ventilsitzfläche relativ zu einer zur Inspirations-Einlassachse orthogonalen Ebene geneigt. In diesem Falle weist der Ventilsitz, auch ein theoretisch möglicher nicht-ebener Ventilsitz, einen dem Inspirations-Einlassabschnitt zugeneigten, angenäherten Näherungsabschnitt und einen vom Inspirations-Einlassabschnitt weg geneigten, weiter entfernt gelegenen Distanzabschnitt auf. Um den Steuerkanal möglichst kurz und damit möglichst verlustfrei halten zu können, verläuft der Steuerkanal bevorzugt näher beim Näherungsabschnitt als beim Distanzabschnitt vom Abzweigort zum Exspirationsventil.

Der Steuerkanal kann wenigstens abschnittsweise als räumlich mit Abstand vom Inspirationskanal oder/und vom Exspirationskanal angeordnetes Kanalbauteil bzw. angeordneter Kanalbauteilabschnitt ausgebildet sein. Dadurch kann, im Gegensatz zur integrierten Bauweise des Steuerkanals mit wenigstens einem Kanal aus Inspirationskanal und Exspirationskanal, jeder dieser Kanäle mit geringst-möglichem Querschnitt und damit die Exspirationsventil-Baugruppe mit möglichst geringem Bauvolumen bereitgestellt werden.

Bevorzugt ist auch bei der vorliegenden Exspirationsventil-Baugruppe daran gedacht, etwaig notwendige Signalleitungen wenigstens abschnittsweise durch die Exspirationsventil-Baugruppe, insbesondere durch wenigstens einen Kanal aus Inspirationskanal und Exspirationskanal, verlaufen zu lassen. Damit eine solche wenigstens eine in einem Kanal geführte Signalleitung sicher aus diesem herausgeführt und mit ihrer kommunizierenden Vorrichtung verbunden werden kann, kann gemäß einer bevorzugten Weiterbildung der vorliegenden Erfindung in einem Bereich zwischen dem Inspirations-Einlassabschnitt und dem Exspirations-Einlassabschnitt oder/und in einem Bereich zwischen dem Inspirations-Einlassabschnitt und einem dem Inspirationsauslass näher als dem Inspirationseinlass gelegenen Inspirations-Auslassabschnitt wenigstens eine Durchlassöffnung ausgebildet sein. Diese wenigstens eine Durchlassöffnung durchsetzt eine den Inspirationskanal oder/und den Exspirationskanal begrenzende Kanalwand.

Die wenigstens eine Durchlassöffnung kann von der wenigstens einen Signalleitung einfach durchsetzt sein. Alternativ kann eine Signalleitung körperlich auf der dem jeweiligen Kanal zugewandten Innenseite der Kanalwand enden. Um das Längsende der Signalleitung sicher zu positionieren kann auf der Innenseite der Kanalwand eine Aufnahmeformation, etwa ein Aufsteckstutzen, ein Einsteckbund oder ein Ring aus gleichgerichtet vom Umfangsrandbereich der Durchgangsöffnung abstehenden Blattfedern, auf bzw. in welchen das Längsende der Signalleitung eingesteckt werden kann, zur Herstellung eines formschlüssigen Aufnahmeeingriffs mit dem Längsende der Signalleitung angeordnet sein.

Ebenso kann auf der Außenseite des betreffenden Kanals eine weitere Aufnahmeformation angeordnet sein, welche in analoger Weise zur Positionierung eines Längsendes einer die Signalleitung außerhalb des die Signalleitung führenden Kanals fortsetzenden weiteren Signalleitung ausgebildet ist. Auch die weitere Aufnahmeformation kann beispielsweise ein Aufsteckstutzen, ein Einsteckbund oder ein Ring aus gleichgerichtet vom Umfangsrandbereich der Durchgangsöffnung abstehenden Blattfedern sein. Die wenigstens eine Aufnahmeformation kann an einem gesonderten Durchführungsbauteil ausgebildet sein, welches in eine Durchgangsöffnung eingesetzt und festgelegt sein kann,

Dann, wenn mehrere Durchgangsöffnungen ausgebildet sind, sind die Durchgangsöffnungen vorzugsweise gleichartig ausgebildet, ebenso eine etwaige Mehrzahl von Durchführungsbauteilen.

Um zu vermeiden, dass die wenigstens eine Signalleitung unnötig gekrümmt werden muss, ist die wenigstens eine Durchlassöffnung möglichst so angeordnet, dass der sich an die Durchlassöffnung anschließende Signalleitungsabschnitt oder die sich anschließende weitere Signalleitung nicht um Kanalbauteile herumgeführt werden müssen. Dies kann dadurch erreicht werden, dass die wenigstens eine Durchlassöffnung in Exspirationsrichtung stromabwärts einer Bezugsebene gelegen ist, welche zur Exspirations-Einlassachse orthogonal orientiert ist und eine vom Ventilsitz eingefasste Öffnungsfläche in gleiche Flächenteile unterteilt.

Weiterhin kann eine unnötige Krümmung der Signalleitung dadurch vermieden werden, dass die wenigstens eine Durchlassöffnung oder/und ein gegebenenfalls in die Durchlassöffnung eingesetztes Durchführungsbauteil längs einer Durchlassachse verläuft, welche parallel ist zur Exspirations-Einlassachse oder/und zu einer Inspirations-Auslassachse, längs welcher sich der Inspirations-Auslassabschnitt erstreckt.

Dabei ist zu berücksichtigen, dass die Signalleitung in der Regel von einem näher als die Exspirationsventil-Baugruppe bei dem Patienten gelegenen Sensor, insbesondere Durchflusssensor, zur Exspirationsventil-Baugruppe verläuft. Die Signalleitung kann eine elektrische Signalleitung sein, welcher elektrische Signale überträgt. Dann ist deren Krümmung in der Regel unkritisch. Gerade bei häufig verwendeten proximalen Differenzdruck-Durchflusssensoren sind zwei Signalleitungen vorgesehen, von welchen jede als hohle Druckleitung einen Atemgasdruck stromaufwärts eines Strömungswiderstands im Durchflusssensor und einen Atemgasdruck stromabwärts des Strömungswiderstands überträgt. Zur möglichst präzisen Druckübertragung ist eine Vermeidung von Krümmungen und Knicken hilfreich.

Grundsätzlich ist es möglich, den Exspirations-Einlassabschnitt und den Inspirations-Auslassabschnitt, welche beide funktionell dem zu beatmenden Patienten zugewandt sind, räumlich getrennt voneinander auszubilden. Gerade in der Notfallmedizin ist jedoch eine möglichst schnelle Rüstung eines Beatmungsgeräts und damit auch der Exspirationsventil-Baugruppe von entscheidendem Vorteil. Daher ist bevorzugt vorgesehen, dass der Exspirations-Einlassabschnitt und der Inspirations-Auslassabschnitt in einem gemeinsamen Multi-Lumen-Endabschnitt ausgebildet sind. Dementsprechend können vorteilhaft die Einlass-Anschlussformation und die Auslass-Verbindungsformation als eine gemeinsame, vorzugsweise einstückige, Anschluss-Verbindungsformation ausgebildet sein. An diese kann ein Multi-Lumen-Schlauch mit einem einzigen Verbindungsvorgang so angeschlossen werden, dass gleichzeitig und mit möglichst nur einem Betätigungsvorgang sowohl die Exspirations-Atemgasleitung an den Exspirationskanal und die Inspirations-Atemgasleitung an den Inspirationskanal angeschlossen werden.

Möglichst kompakt kann die Exspirationsventil-Baugruppe dadurch bereitgestellt werden, dass der Inspirations-Einlassabschnitt, der Exspirations-Einlassabschnitt und der Inspirations-Auslassabschnitt durch ein einstückiges Kanalbauteil realisiert sind. Bevorzugt ist das einstückige Kanalbauteil im Kunststoff-Spritzgussverfahren hergestellt. Das einstückige Kanalbauteil weist, vorzugsweise ebenso einstückig daran ausgebildet, die Einlass-Anschlussformation, die Einlass-Verbindungsformation und die Auslass-Verbindungsformation auf, bevorzugt jeweils als Verbindungsstutzen oder/und Verbindungsbuchse.

Die vorliegende Erfindung betrifft außerdem eine Atemgas-Leitungsbaugruppe, umfassend eine Exspirationsventil-Baugruppe, wie sie oben beschrieben und weitergebildet ist, eine Exspirations-Atemgasleitung, eine Inspirations-Atemgasleitung sowie einen Durchflusssensor.

Die Exspirations-Atemgasleitung weist bevorzugt an ihrem distalen Längsende eine distale exspiratorische Kopplungsformation auf, welche zur Herstellung einer eine exspiratorische Atemgasströmung leitenden Verbindung mit der Einlass-Anschlussformation des Exspirationskanals der Exspirationsventil-Baugruppe ausgebildet ist. Ebenso weist die Exspirations-Atemgasleitung an ihrem proximalen Längsende eine proximale exspiratorische Kopplungsformation auf, welche zur Herstellung einer eine exspiratorische Atemgasströmung leitenden Verbindung mit einer exspiratorischen Ausgabe-Anschlussformation am distalen Längsende des Durchflusssensors ausgebildet ist. Weiterhin weist die Inspirations-Atemgasleitung an ihrem distalen Längsende eine distale inspiratorische Kopplungsformation auf, welche zur Herstellung einer eine inspiratorische Atemgasströmung leitenden Verbindung mit der Auslass-Verbindungsformation des Inspirationskanals der Exspirationsventil-Baugruppe ausgebildet ist. Schließlich weist die Inspirations-Atemgasleitung an ihrem proximalen Längsende eine proximale inspiratorische Kopplungsformation auf, welche zur Herstellung einer eine inspiratorische Atemgasströmung leitenden Verbindung mit einer inspiratorischen Eingabe-Anschlussformation am distalen Längsende des Durchflusssensors ausgebildet ist.

Zur Vermeidung einer unnötig großen Anzahl unterschiedlicher Leitungen oder Schläuche, an welchen sich Rettungspersonal oder ärztliches Personal im Einsatz verheddern könnte, ist bevorzugt in wenigstens einer Atemgasleitung aus Exspirations- und Inspirations-Atemgasleitung wenigstens eine Signalleitung aufgenommen, welche dazu ausgebildet ist, eine Erfassungsinformation des Durchflusssensors vom proximalen zum distalen Längsende der wenigstens einen Atemgasleitung zu übertragen. Es handelt sich dabei um die oben bereits erwähnte wenigstens eine Signalleitung.

In einer solchen Atemgas-Leitungsbaugruppe ist bevorzugt das oben beschriebene Einwegventil im Inspirationskanal der Exspirationsventil-Baugruppe das einzige Einwegventil in der inspiratorischen Atemgasströmung vom Inspirationseinlass bis zum proximalen Ende des Durchflusssensors. Eine weitere Ventilanordnung im Inspirationskanal bzw. in der gesamten Inspirations-Atemgasleitung ist nicht funktionsnotwendig.

Ebenso ist bevorzugt das Exspirationsventil die einzige Ventilanordnung im Exspirationskanal oder besonders bevorzugt in der gesamten Exspirations-Atemgasleitung.

Wie oben bereits dargelegt wurde, kann wenigstens eine der wenigstens einen Signalleitung wenigstens eine der wenigstens einen Durchlassöffnung durchsetzen oder in einem Aufnahmeeingriff einer Aufnahmeformation zur Aufnahme des distalen Endes der wenigstens einen Signalleitung enden. Mögliche Aufnahmeformationen sind oben bereits genannt.

Zur weiteren Vermeidung einer unnötig großen Anzahl an Leitungen, insbesondere Schläuchen, welche das Risiko erhöht, dass sich im Betriebsbereich der Atemgas-Leitungsbaugruppe tätiges Personal verheddert und eine Beatmung eines Patienten gefährdet oder sogar beendet, sind bevorzugt die Exspirations-Atemgasleitung und die Inspirations-Atemgasleitung an einem gemeinsamen Multi-Lumen-Leitungsbauteil ausgebildet. Zwar kann das Multi-Lumen-Leitungsbauteil, etwa als Multi-Lumen-Atemgasschlauch, auch an räumlich voneinander getrennte Formationen aus Einlass-Anschlussformation und Auslass-Verbindungsformation angeschlossen sein. Bevorzugt ist das Multi-Lumen-Leitungsbauteil jedoch an den oben genannten Multi-Lumen-Endabschnitt der Exspirationsventil-Baugruppe angeschlossen.

Wenngleich dass Multi-Lumen-Leitungsbauteil als Bi-Lumen-Leitungsbauteil zwei koaxiale, vorzugsweise konzentrische, Schläuche aufweisen kann, ist zur Bereitstellung etwa gleicher Querschnittsflächen in der Inspirations- wie in der Exspirations-Atemgasleitung mit etwa gleicher Wandfläche pro Streckeneinheit bevorzugt, wenn die Exspirations- und Inspirations-Atemgasleitung durch ein längs des Leitungsbauteils sowie längs dessen Innendurchmessers verlaufendes Septum in dem Leitungsbauteil voneinander abgegrenzt sind. Bevorzugt ist das Leitungsbauteil ein Schlauch.

Um die beiden Atemgasleitungen aus Exspirations- und Inspirations-Atemgasleitung möglichst gleichmäßig durch die Aufnahme von Signalleitungen zu belasten, sind bevorzugt im Exspirationslumen und im Inspirationslumen gleich viele Signalleitungen aufgenommen, bevorzugt jeweils genau eine.

An ihrem proximalen Längsende kann die Atemgas-Leitungsbaugruppe, insbesondere das Leitungsbauteil, besonders bevorzugt das Multi-Lumen-Leitungsbauteil, ein proximales Kopplungsbauteil aufweisen, mit welchem das Leitungsbauteil, insbesondere das Multi-Lumen-Leitungsbauteil, verbunden ist. Das Kopplungsbauteil weist bevorzugt an seinem distalen Längsende eine Exspirations-Verbindungsformation zur Herstellung einer eine exspiratorische Atemgasströmung leitenden Verbindung mit der Exspirations-Atemgasleitung, insbesondere mit dem Exspirationslumen des Multi-Lumen-Leitungsbauteils, und eine Inspirations-Verbindungsformation zur Herstellung einer eine inspiratorische Atemgasströmung leitenden Verbindung mit der Inspirations-Atemgasleitung, insbesondere mit dem Inspirationslumen des Multi-Lumen-Leitungsbauteils, auf. Das Kopplungsbauteil weist an seinem proximalen Längsende bevorzugt eine Kopplungsformation auf, welche sowohl die proximale exspiratorische Kopplungsformation als auch die proximale inspiratorische Kopplungsformation ist. DE102019121712 A1 offenbart eine Atemgas-Ventilbaugruppe für eine Beatmungsleitungsanordnung einer Beatmungsvorrichtung zur wenigstens unterstützenden künstlichen Beatmung eines Patienten.

Die vorliegende Erfindung wird nachfolgend anhand der beiliegenden Zeichnungen näher erläutert. Es stellt dar:
- Fig. 1A: eine Explosions-Seitenansicht eines distalen Endes einer erfindungsgemäßen Ausführungsform einer Atemgas-Leitungsbaugruppe mit einer erfindungsgemäßen Ausführungsform einer Exspirationsventil-Baugruppe,
- Fig. 1B: eine Explosions-Seitenansicht eines proximalen Endes der Atemgas-Leitungsbaugruppe von Figur 1A,
- Fig. 2A: eine Seitenansicht des distalen Endes der Atemgas-Leitungsbaugruppe von Figur 1A in zusammengebautem Zustand bei Betrachtung längs des Pfeils IIA in den Figuren 3A und 4,
- Fig. 2B: eine Seitenansicht des proximalen Endes der Atemgas-Leitungsbaugruppe von Figur 1B in zusammengebauten Zustand bei Betrachtung längs des Pfeils IIB in Figur 3B,
- Fig. 3A: eine Unteransicht des proximalen Endes der Atemgas-Leitungsbaugruppe von Figur 2A längs des Pfeils IIIA in den Figuren 2A und 4,
- Fig. 3B: eine Unteransicht des distalen Endes der Atemgas-Leitungsbaugruppe von Figur 2B längs des Pfeils IIIB in Figur 2B,
- Fig. 4: eine Aufrissansicht der Atemgas-Leitungsbaugruppe bei Betrachtung längs des Pfeils IV in den Figuren 2B und 3B,
- Fig. 5: eine Längsschnittansicht eines einstückigen Kanalbauteils der Exspirationsventil-Baugruppe von Figur 4 längs der Schnittebene V-V von Figur 4.

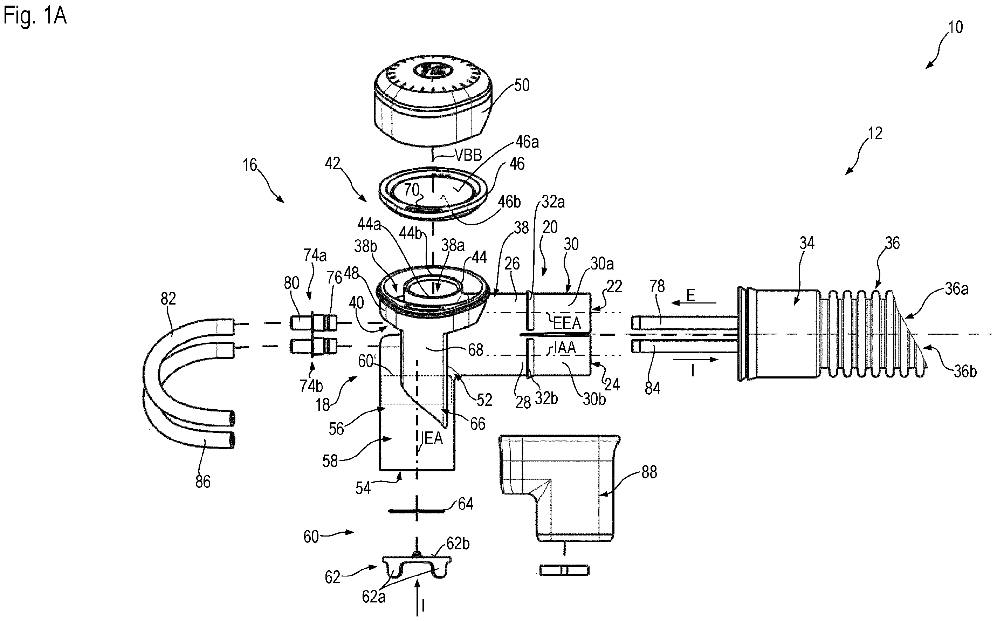

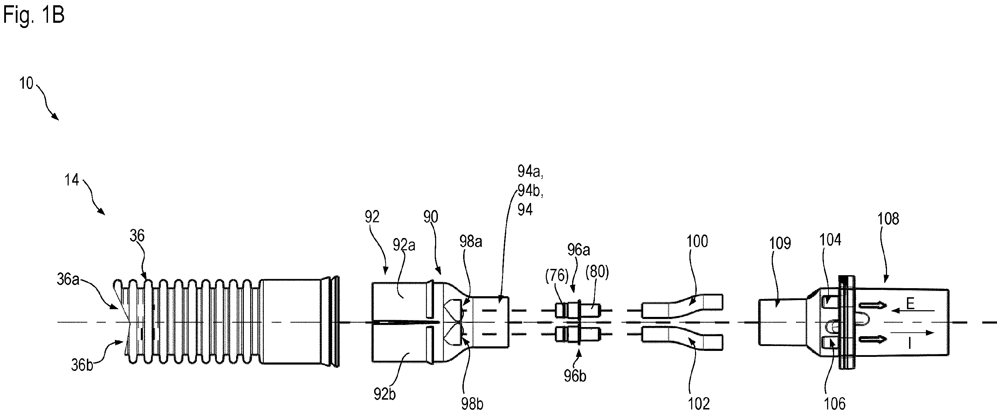

In den Figuren 1A bis 3B ist eine erfindungsgemäße Ausführungsform einer Atemgas-Leitungsbaugruppe allgemein mit 10 bezeichnet. In den Figuren 1A, 2A und 3A ist der distale Längsendbereich 12 der Atemgas-Leitungsbaugruppe 10 dargestellt, in den Figuren 1B, 2B und 3B deren proximaler Längsendbereich 14.

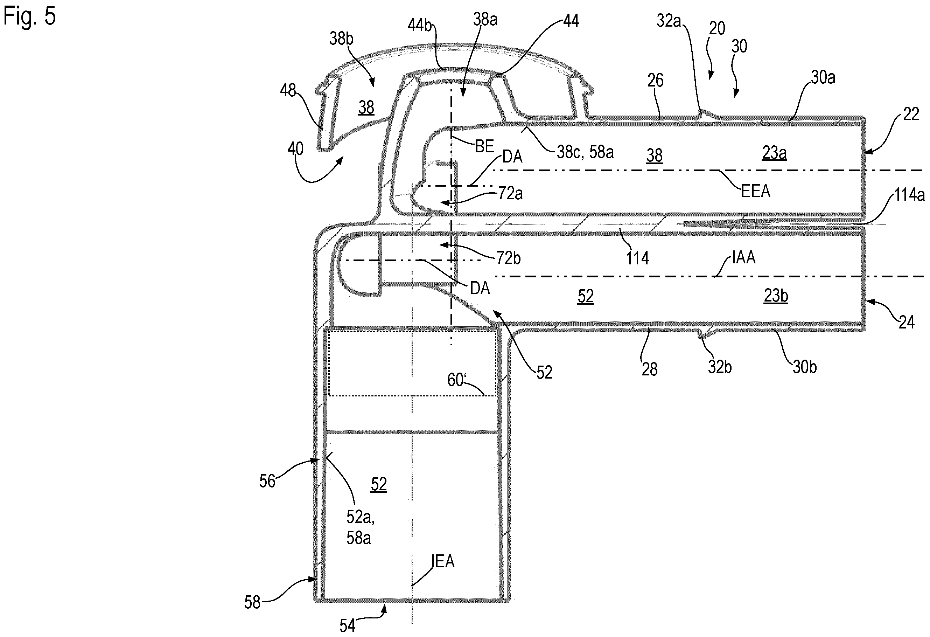

Das distale, also vom beatmeten Patienten weiter entfernt gelegene Längsende 12 der Atemgas-Leitungsbaugruppe 10 umfasst eine Exspirationsventil-Baugruppe 16 mit einem einstückig aus Kunststoff spritzgegossenen Kanalbauteil 18. Das Kanalbauteil 18 in Alleinstellung ist im Längsschnitt in Figur 5 gezeigt.

Das Kanalbauteil 18 umfasst einen Multi-Lumen-Endabschnitt 20, welcher in seinem in Figur 1A oberen Bereich einen Exspirationseinlass 22 und in seinem unteren Bereich einen Inspirationsauslass 24 aufweist. An den Exspirationseinlass 22 schließt sich in Exspirationsrichtung E ein geradliniger Exspirations-Einlassabschnitt 26 an, welcher sich längs einer Exspirations-Einlassachse EEA erstreckt. An den Inspirationsauslass 24 schließt sich entgegen der Inspirationsrichtung I ein geradliniger Inspirations-Auslassabschnitt 28 an, welcher sich längs einer Inspirations-Auslassachse IAA erstreckt. Die Exspirations-Einlassachse EEA und die Inspirations-Auslassachse IAA sind im dargestellten Ausführungsbeispiel zueinander parallel, ebenso der Exspirations-Einlassabschnitt 26 und der Inspirations-Auslassabschnitt 28.

Der Multi-Lumen-Endabschnitt 20 weist eine gemeinsame Anschluss-Verbindungsformation 30 auf, welche in ihrem in Figur 1A oberen Bereich als Einlass-Anschlussformation 30a und in ihrem in Figur 1A unteren Bereich als Auslass-Verbindungsformation 30b wirkt. Radialvorsprünge 32a und 32b, welche als Endanschläge für eine kooperierende Anschluss-Verbindungsgegenformation 34 des Multi-Lumen-Schlauchs als dem Multi-Lumen-Leitungsbauteil 36 der Atemgas-Leitungsbaugruppe 10 wirken, zeigen das Längsende der Einlass-Anschlussformation 30a sowie der Auslass-Verbindungformation 30b an.

Der Multi-Lumen-Schlauch 36 kann mit seiner Anschluss-Verbindungsgegenformation 34 auf die Anschluss-Verbindungformation 30 des Multi-Lumen-Endabschnitts 20 aufgeschoben werden, um eine Strömungsverbindung zwischen dem Multi-Lumen-Schlauch 36 und dem Kanalbauteil 18 herzustellen. Die Anschluss-Verbindungsgegenformation 34 kann reibschlüssig oder formschlüssig, beispielsweise durch Bajonettverschluss oder durch eine federnde Verrastung oder durch andere Verschlussmittel, lösbar an der Anschluss-Verbindungformation 30 gehalten sein.

In der Exspirationsventil-Baugruppe ist ein Exspirationskanal 38 ausgebildet, welcher vom Exspirationseinlass 22 bis zu einem Exspirationsauslass 40 verläuft. Der Exspirations-Einlassabschnitt 26 ist Teil des Exspirationskanals 38.

Im Exspirationskanal 38 ist in Exspirationsrichtung E zwischen dem Exspirationseinlass 22 und dem Exspirationsauslass 40 ein Exspirationsventil 42 in Gestalt eines Membranventils angeordnet. In Figur 1A ist der ringförmige, ebene, jedoch geneigte Ventilsitz 44 zu erkennen, an welchem in Exspirationsrichtung E ein einlassseitiger Exspirationskanalabschnitt 38a endet, und von welchem in Exspirationsrichtung E ein auslassseitiger Exspirationskanalabschnitt 38b ausgeht. Die Exspirationskanalabschnitte 38a und 38b sind durch das Exspirationsventil 42 voneinander getrennt. Das Exspirationsventil 42 weist hierfür einen Ventilkörper 46 in Gestalt einer Membran auf, welche an der Kanalwand 48 des auslassseitigen Exspirationskanalabschnitts 38b durch ein Deckelbauteil 50 gehalten ist. Der Membranventilkörper 46 kann zwischen der Kanalwand 48 und dem Deckelbauteil 50 geklemmt sein.

Strömt exspiratorisches Atemgas in Exspirationsrichtung E durch den einlassseitigen Exspirationskanalabschnitt 38a an den Membranventilkörper 46, wird dieser durch den sich aufbauenden Druck entgegen der durch die Materialelastizität des Membranventilkörpers 46 bewirkten Vorspannkraft vom ringförmigen Ventilsitz 44 abgehoben und das exspiratorische Atemgas kann von dem einlassseitigen Exspirationskanalabschnitt 38a in den diesen radial außen umgebenden auslassseitigen Exspirationskanal 38b überströmen.

In der Exspirationsventil-Baugruppe 16, insbesondere im einstückigen Kanalbauteil 18, ist ein Inspirationskanal 52 ausgebildet, welcher am deutlichsten in Figur 5 zu erkennen ist. Dieser reicht in Inspirationsrichtung I von einem Inspirationseinlass 54 bis zum Inspirationsauslass 24.

An den Inspirationseinlass 54 schließt sich in Inspirationsrichtung I ein geradliniger Inspirations-Einlassabschnitt 56 als Teil des Inspirationskanals 52 an, welcher längs einer Inspirations-Einlassachse IEA verläuft, die im dargestellten Beispiel mit der Inspirations-Auslassachse IAA einen Winkel von 90° einschließt. Ein den Inspirationseinlass 54 aufweisender Bereich des Inspirations-Einlassabschnitts 56 ist als Einlass-Verbindungsformation 58 ausgebildet, welche der Herstellung einer inspiratorisches Atemgas in Inspirationsrichtung I leitenden Verbindung dient, mit der das inspiratorische Atemgas von einer in den Figuren nicht dargestellten Atemgasquelle, etwa von einem Beatmungsgerät, in den Inspirationskanal 52 eingeleitet werden kann. Die Einlass-Verbindungsformation 58 kann als Aufsteckbuchse ausgebildet sein, welche auf einen Anschlussstutzen an der Atemgasquelle, diesen radial außen umgebend, aufsteckbar ist. Alternativ kann die Einlass-Verbindungsformation 58 als Anschlussstutzen ausgebildet sein, welcher in eine Buchse an der Atemgasquelle einsteckbar ist.

In den Inspirations-Einlassabschnitt 56 ist ein Einwegventil 60 eingeführt, welches ein Basisbauteil 62 und einen daran gehalterten elastomeren scheibenförmigen Ventilkörper 64 aufweist. Das Einwegventil 60 ist mit einer Innenwand 52a (siehe Figur 5) des Inspirationskanals 52 bevorzugt durch Ultraschallschweißen von Befestigungslaschen 62a des Basisbauteils 62 mit der Innenwand 52a verschweißt und somit lagegesichert. Der Ventilkörper 64 ist in einem zentralen Bereich formschlüssig am Basisbauteil 62 gehaltert und kann bei Anströmung in Inspirationsrichtung I mit seinem Umfangsrand Bereich von einem am Basisbauteil 62 ausgebildeten Ventilsitz 62b abgehoben werden. Bei Anströmung entgegen der Inspirationsrichtung I wird der Ventilkörper 64 gegen den Ventilsitz 62b gedrückt und verschließt so den Inspirationskanal 52.

Analog zum Inspirationskanal 52 ist der Exspirationskanal 38 durch eine Kanalwand 38c begrenzt. Die Kanalwand 48 des auslassseitigen Exspirationskanalabschnitt 38b ist Teil der Kanalwand 38c.

Ein strichliniertes Rechteck 60' zeigt in Figur 1 die Lage des Einwegventils 60 im Kanalbauteil 18 im fertig montierten Zustand an. In Figur 1 ist der scheibenförmige Ventilkörper 64 derart orientiert, dass die Erstreckungsebene der Ventilkörperscheibe orthogonal zur Zeichenebene von Figur 1 gelegen ist.

Von dem Inspirationskanal 52 zweigt von einem Abzweigort 66, welcher in Inspirationsrichtung I stromaufwärts des eingebauten Ventilkörpers 64 des Einwegventils 60 gelegen ist, ein Steuerkanal 68 ab, welcher mittels einer Öffnung 70 im Membranventilkörper 46 den Membranventilkörper 46 durchsetzt und in einer vom Deckelbauteil 50 auf der vom Ventilsitz 44 des Exspirationsventils 42 abgewandten Seite gelegenen Kammer endet. Durch den Steuerkanal 68 ist somit unabhängig vom Betriebszustand des Einwegventils 60 stets inspiratorisches Atemgas auf die vom Ventilsitz 44 abgewandte Seite des Membranventilkörpers 46 leitbar, sodass während eines Inspirationsvorgangs durch das inspiratorische Atemgas das Exspirationsventil 42 sicher in seiner Sperrstellung gehalten werden kann, in welcher der auf dem Ventilsitz 44 aufliegende Membranventilkörper 46 eine Durchströmung des Exspirationskanals 38 sperrt. Der Steuerkanal 68 liegt abschnittsweise baulich sowohl außerhalb des Inspirationskanals 52 als auch außerhalb des Exspirationskanals 38 und hat wenigstens längs eines Abschnitts keinen Wandabschnitt mit dem Inspirationskanal 52 oder dem Exspirationskanal 38 derart gemein, dass der gemeinsame Wandabschnitt auf einer Seite den Steuerkanal 68 und auf seiner entgegengesetzten Seite einen Kanal aus Inspirationskanal 52 und Exspirationskanal 38 begrenzen würde. Lediglich im Bereich der Kanalwand 48 des auslassseitigen Exspirationskanalabschnitts 38b existiert ein Wandabschnitt, der sowohl einen Abschnitt des Steuerkanals 68 als auch einen Abschnitt des auslassseitigen Exspirationskanalabschnitts 38b begrenzt.

Um während eines Inspirationsvorgangs das Exspirationsventil sicher geschlossen halten zu können, ist die inspiratorische Membranfläche 46a des Membranventilkörpers 46, welche von inspiratorischem Atemgas benetzbar ist, größer als die entgegengesetzte exspiratorische Membranfläche 46b, welche bei geschlossenem Exspirationsventil 46 innerhalb des Ventilsitzes 44 gelegen ist und von exspiratorischem Atemgas aus dem einlassseitigen Exspirationskanalabschnitt 38a anströmbar ist. Im dargestellten bevorzugten Fall ist die inspiratorische Membranfläche 46a 1,8 mal größer als die exspiratorische Membranfläche 46b.

Das Kanalbauteil 58 weist in Figur 5 erkennbare Durchgangsöffnungen 72a im Exspirationskanal 38 und 72b im Inspirationskanal 52 auf. Die jeweils längs einer zur Exspirations-Einlassachse EEA und zur Inspirations-Auslassachse IAA parallelen Durchlassachse DA verlaufenden Durchgangsöffnungen 72a und 72b durchsetzen die Kanalbauteilwand 58a des Kanalbauteils 58, welche sowohl den Inspirationskanal 52 wie auch den Exspirationskanal 38 begrenzt.

In die Durchgangsöffnungen 72a und 72b sind in Figur 1 dargestellte und gesondert vom Kanalbauteil 18 ausgebildete, sich jeweils längs der zugeordneten Durchlassachse DA erstreckende Durchführungsbauteile 74a bzw. 74b eingebaut. Da die Durchführungsbauteile 74a und 74b identisch sind, genügt die Beschreibung nur des Durchführungsbauteils 74a, welche auch für das andere Durchführungsbauteil 74b gilt.

Das Durchführungsbauteil 74a weist auf seiner in den Exspirationskanal 38, also in den Innenbereich des Kanalbauteils 18, weisenden Seite eine Aufnahmeformation 76 auf, mit welcher eine im Exspirationslumen 36a des Multi-Lumen-Schlauchs 36 geführte erste Signalleitung 78 formschlüssig verbindbar ist. Die Aufnahmeformation 76 kann ein die Signalleitung 78 umgebender Bund, Federring oder ein Aufsteckstutzen sein, auf welchen die Signalleitung 78 aufsteckbar ist.

Auf seiner entgegengesetzten Seite weist das Durchführungsbauteil 74a eine weitere Aufnahmeformation 80 auf, mit welcher eine weitere Signalleitung 82 formschlüssig verbindbar ist. Auch die weitere Aufnahmeformation 80 kann ein Bund, Federring oder ein Aufsteckstutzen sein.

In dem Multi-Lumen-Schlauch 36 ist im Inspirationslumen 36b eine zweite Signalleitung 84 geführt, welche analog zum oben beschriebenen Durchführungsbauteil 74a mittels des Durchführungsbauteils 74b auf der Außenseite des Kanalbauteils 18 durch eine noch weitere Signalleitung 86 fortgesetzt sein kann.

Die Signalleitungen 78 und 84 sowie die weiteren Signalleitungen 82 und 86 übertragen als hohle Leitungen eine Druckinformation von einem weiter unten beschriebenen Durchflusssensor 108 im proximalen Längsendbereich 14 der Atemgas-Leitungsbaugruppe 10. Die weiteren Signalleitungen 82 und 86 können in einen Stecker 88 münden, mit welchem einfach und sicher eine die Druckinformation übertragende Verbindung mit einem Drucksensor in einem Beatmungsgerät herstellbar ist.

In Figur 1B ist das proximale, also im Betrieb näher beim beatmeten Patienten gelegene, Längsende 14 der Atemgas-Leitungsbaugruppe 10 gezeigt. Der Multi-Lumen-Schlauch 36 ist an seinem distalen Längsende identisch zum oben bereits beschriebenen proximalen Längsende aufgebaut, sodass zur Erläuterung des distalen Längsendes auf die Beschreibung des proximalen Längsendes des Multi-Lumen-Schlauchs 36 verwiesen wird.

An das proximale Längsende des Multi-Lumen-Schlauchs 36 ist ein als gesondertes Bauteil ausgebildetes proximales Kopplungsbauteil 90 angekoppelt, welches an seinem distalen Längsende eine gemeinsame Anschluss-Verbindungsformation 92 aufweist, die der oben bereits beschriebenen Anschluss-Verbindungsformation 30 entspricht, auf deren Beschreibung auch zur Erläuterung der Anschluss-Verbindungsformation 92 verwiesen wird. Die gemeinsame Anschluss-Verbindungsformation 92 umfasst eine in Figur 1B obere Exspirations-Verbindungsformation 92a, welche der Einlass-Verbindungsformation 30a am Kanalbauteil 18 entspricht, und umfasst eine in Figur 1B untere Inspirations-Verbindungsformation 92b, welche der Auslass-Verbindungsformation 30b am Kanalbauteil 18 entspricht. Das proximale Kopplungsbauteil 90 ist in seiner Funktion ein Y-Verbindungsbauteil, denn es führt das Exspirationslumen und das Inspirationslumen an seinem proximalen Längsende zu einer gemeinsamen Kopplungsformation 94 zusammen, welche sowohl eine proximale exspiratorische Kopplungsformation 94a als auch eine proximale inspiratorische Kopplungsformation 94b ist. Die gemeinsame Kopplungsformation 94 wird daher sowohl von inspiratorischem wie auch von exspiratorischem Atemgas durchströmt, wobei die genannten Atemgase abhängig von den Stellungen des Exspirationsventils 42 und des Einwegventils 60 in den ihnen zugeordneten Lumina aus Exspirationslumen und Inspirationslumen strömen. Die gemeinsame Kopplungsformation 94 ist mit einer Eingabe-Anschlussformation 109 am distalen Längsende eines Durchflusssensors 108 verbindbar, beispielsweise steckbar.

Die im Exspirationslumen 36a verlaufende erste Signalleitung 78 und im Inspirationslumen 36b verlaufende zweite Signalleitung 84 sind in Figur 1B nicht gezeigt. Sie sind jedoch vorhanden und münden erneut in Durchführungsbauteilen 96a und 96b, welche mit den zuvor beschriebenen Durchführungsbauteilen 74a und 74b identisch sind. Die Durchführungsbauteile 96a und 96b sind jeweils in einer die Wandung des proximalen Kopplungsbauteils 90 durchsetzenden Durchgangsöffnung 98a bzw. 98b angeordnet. Außerhalb des Kopplungsbauteils 90 werden die Signalleitungen 78 und 84 durch weitere proximale Signalleitungen 100 und 102 fortgesetzt, welche an Anschlussstutzen 104 bzw. 106 eines Differenzdruck-Durchflusssensors 108 enden. Die Signalleitungen 100 und 102 sowie 80 bzw. 84 und weiter 82 bzw. 86 übertragen als Schlauchleitungen die Drücke beiderseits eines nicht näher dargestellten, aber an sich bekannten Strömungswiderstands im Inneren des Differenzdruck-Durchflusssensors 108 zu einem Drucksensor im Beatmungsgerät, welche aus den übertragenen Druckinformationen die den proximalen Differenzdruck-Durchflusssensor 108 bidirektional durchströmenden Atemgasströme während der Inspiration und der Exspiration ermittelt.

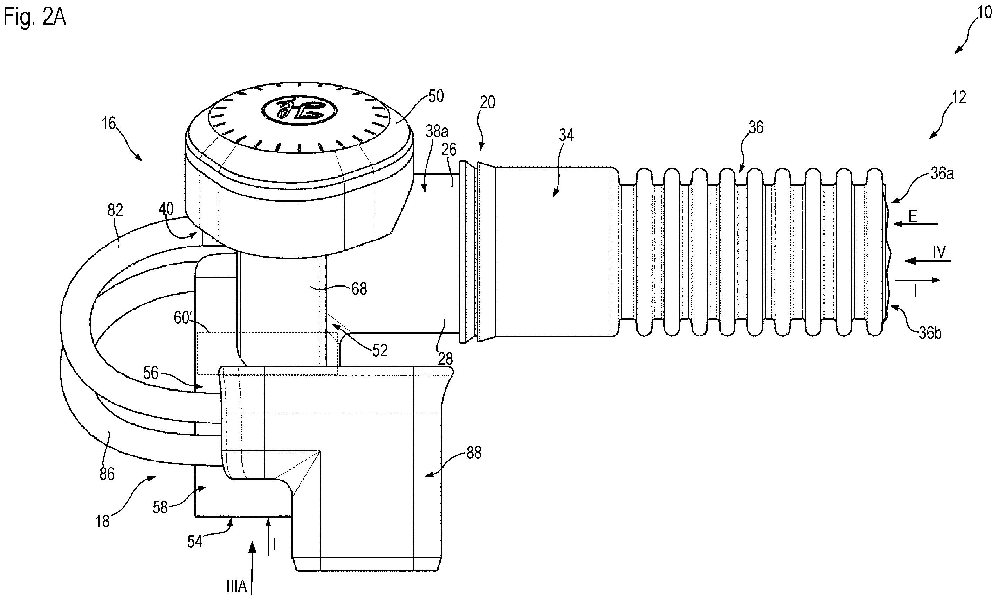

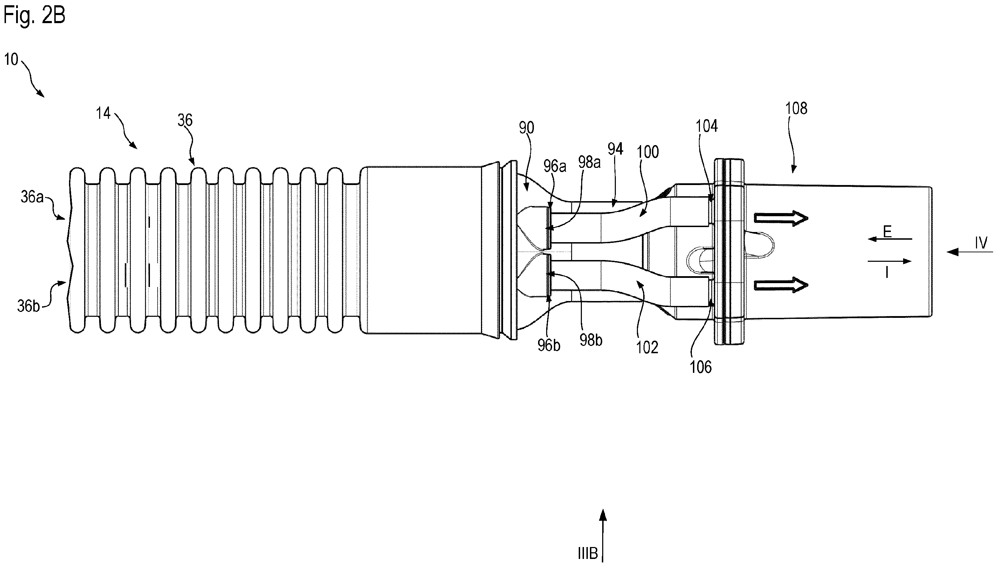

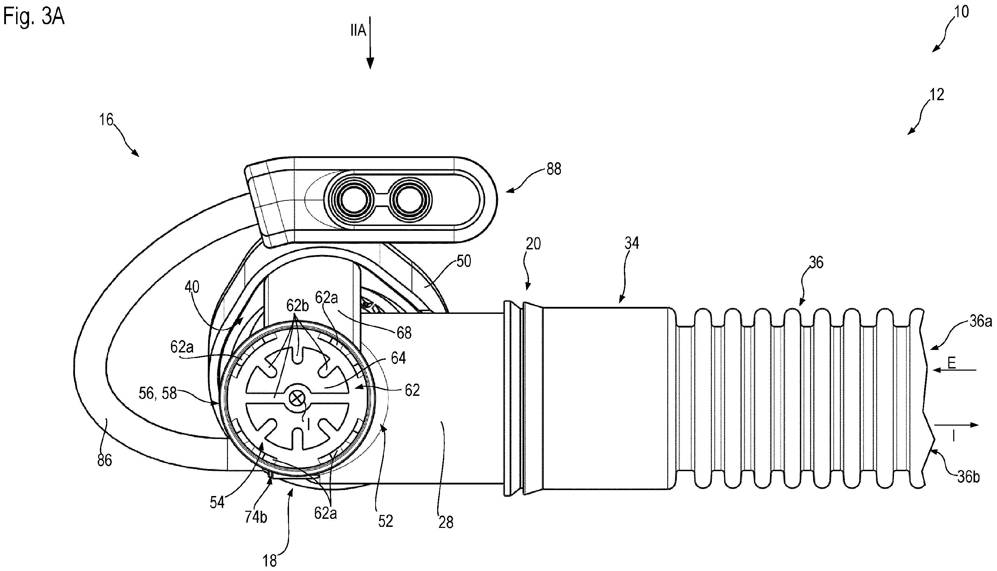

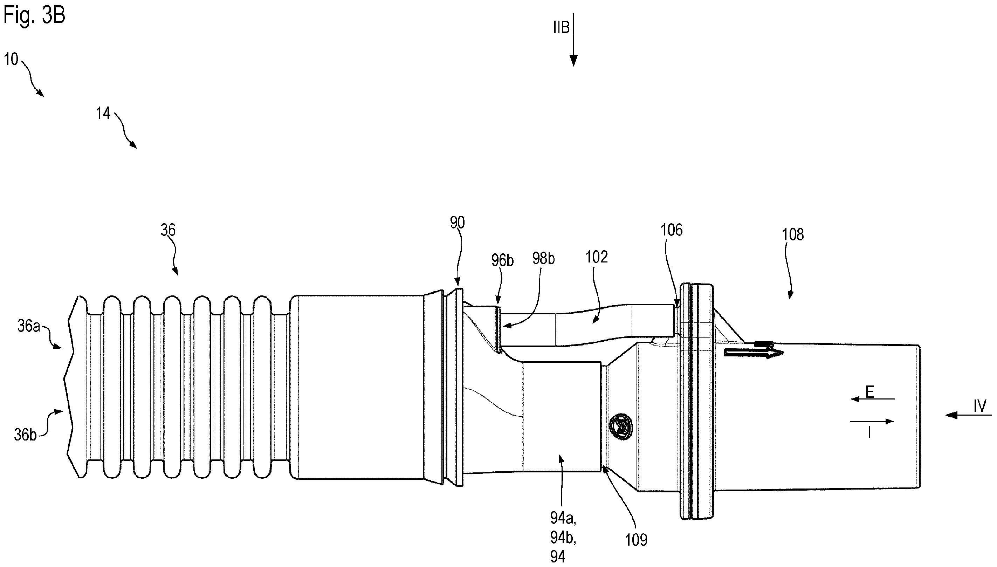

In den Figuren 2A und 3A ist das distale Längsende der Atemgas-Leitungsbaugruppe 10 im montierten Zustand aus den in der obigen Figurenaufzählung genannten und in den Figuren dargestellten Blickrichtungen dargestellt. In den Figuren 2B und 3B gilt das entsprechende für das proximale Längsende der Atemgas-Leitungsbaugruppe 10.

Dabei ist in der Unteransicht von Figur 3A die Struktur des Basisbauteils 62 mit den Befestigungslaschen 62a und dem Ventilsitz 62b des Einwegventils 60 sowie der sich bei Betrachtung der Figur 3A dahinter befindliche Ventilkörper 64 zu erkennen. Der besseren Übersichtlichkeit wegen sind nicht alle Strukturen des Ventilsitzes 62b mit einem Bezugszeichen versehen.

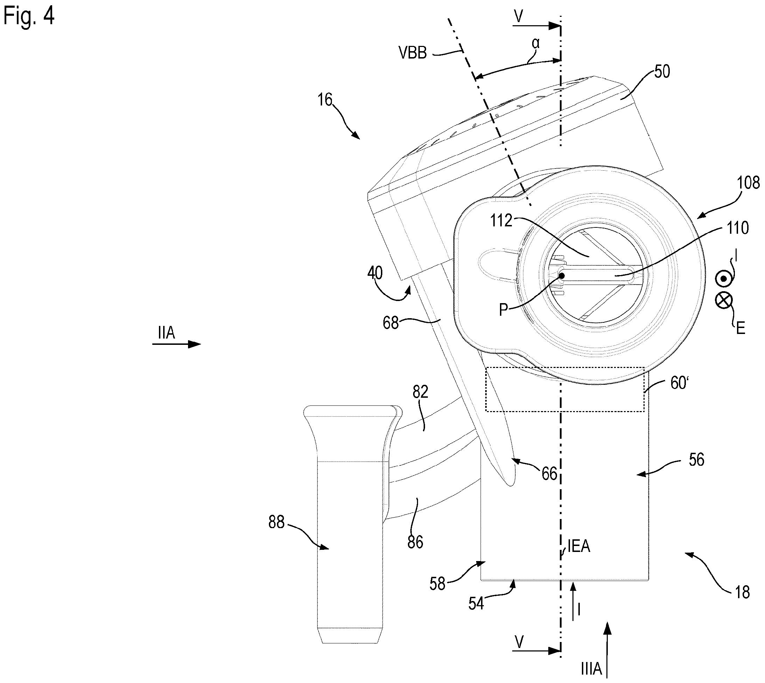

In Figur 4 ist die Atemgas-Leitungsbaugruppe 10 längs einer zur Zeichenebene von Figur 4 orthogonalen Parallelen P zur Exspirations-Einlassachse EEA sowie zur Inspirations-Auslassachse IAA von dem proximalen Ende des Differenzdruck-Durchflusssensors 108 aus betrachtet. Abgesehen davon, dass man in Figur 4 in die sowohl von inspiratorischem als auch von exspiratorischem Atemgas durchströmte proximale Öffnung des Durchflusssensors 108 blickt und dort ein sich orthogonal zur Zeichenebene von Figur 4 verlaufendes Strömungsleitelement 110 sowie ein dahinter gelegenes, zu Zeichenebene der Fig. 4 paralleles folienartiges Strömungswiderstandsbauteil 112 mit strömungsabhängig variablem Strömungswiderstand erkennt, zeigt Figur 4 auch die Neigung des Deckelbauteils 50 bezüglich der Inspirations-Einlassachse IEA um die Parallele P. Der Neigungswinkel α beträgt im dargestellten Beispiel zwischen 20 und 30°, vorzugsweise zwischen 22 und 26°, besonders bevorzugt 24° oder 25°.

Durch diese Konstruktion ist auch die Ventilbewegungsbahn VBB, längs welcher sich der Membranventilkörper 46 zur Bewegung in seine Durchlassstellung vom Ventilsitz 44 abhebt und zur Rückkehr in seiner Sperrstellung auf den Ventilsitz 44 zu bewegt, um den Neigungswinkel α geneigt. Hierdurch wrd eine Exspirationsventil-Baugruppe 16 erhalten, an welcher selbst im hektischen Betrieb an Unfallstellen und in Rettungssituationen die Schwerkraft zur Belastung des Membranventilkörpers 64 in Richtung auf den Ventilsitz 44 zu beiträgt und somit eine Art Vorspannung des Exspirationsventils 42 in die Sperrstellung unterstützt. Diese Belastung durch die Schwerkraft tritt zur Vorspannung des Exspirationsventils 42 in die Sperrstellung aufgrund der Konstruktion und der Materialelastizität des Membranventilkörpers 64 noch hinzu.

Der Steuerkanal 68 verläuft bevorzugt parallel zur Ventilbewegungsbahn VBB.

Durch die Neigung der Ventilbewegungsbahn VBB und damit des Deckelbauteils 50 weist das Deckelbauteil 50, vor allem jedoch das Exspirationsventil 42 und insbesondere der zur Ventilbewegungsbahn VBB orthogonal verlaufende Ventilsitz 44, einen in Figur 1 gezeigten Näherungsabschnitt 44a auf, welcher näher beim Inspirationseinlass 54 gelegen ist, und einen Distanzabschnitt 44b auf, welcher weiter vom Inspirationseinlass 54 entfernt gelegen ist. Der Distanzabschnitt 44b und der Näherungsabschnitt 44a liegen einander bezüglich der Ventilbewegungsbahn VBB diametral gegenüber. Um einen besonders kurzen Steuerkanal 68 realisieren zu können, befindet sich dieser auf der Seite des Näherungsabschnitt 44a.

In Figur 5 ist ein Längsschnitt durch das Kanalbauteil 18 längs der in Figur 4 gezeigten Schnittebene V-V gezeigt. Dort ist ebenfalls der Distanzabschnitt 44b des Ventilsitzes 44 zu erkennen.

Figur 5 zeigt ebenfalls die Durchgangsöffnungen 72a und 72b sowie das orthogonal zur Zeichenebene von Figur 5 orientierte im Wesentlichen ebene Septum 114, welches den Multi-Lumen-Endabschnitt 20 im Kanalbauteil 18 in ein Exspirationslumen 23a und ein Inspirationslumen 23b unterteilt. Die Lumina Exspirationslumen 23a und Inspirationslumen 23b setzen sich im fertig montierten Zustand der Atemgas-Leitungsbaugruppe 10 im Multi-Lumen-Schlauch 36 im Exspirationslumen 36a bzw. im Inspirationslumen 36b fort. Auch der Multi-Lumen-Schlauch 36 ist durch ein im Wesentlichen ebenes, ihn diametral durchsetzendes Septum in die zwei Lumina 36a und 36b unterteilt. Eine im Septum 114 ausgebildete Nut 114a dient der gasdichten Verbindung der beiden Septa des Multi-Lumen-Endabschnitt 20 und des Multi-Lumen-Schlauchs 36.

In Fig. 5 markiert das Bezugszeichen BE eine zur Zeichenebene der Fig. 5 orthogonale Bezugsebene BE, welche eine vom Ventilsitz 44 umgebene Öffnungsfläche in beiderseits der Bezugsebene BE gelegene gleichgroße Flächenanteile unterteilt. Die Durchlassöffnungen 72a und 72b liegen in Exspirationsrichtung E stromabwärts der Bezugsebene BE. Dadurch können die Signalleitungen 78 und 84 so lange wie möglich so wenig gekrümmt wie möglich verlaufen.

## Patentansprüche

1. Exspirationsventil-Baugruppe (16) für eine Beatmungsvorrichtung zur künstlichen Beatmung eines Patienten, umfassend
- einen Exspirationskanal (38), welcher an einem Ende einen Exspirationseinlass (22) zum Einleiten einer exspiratorischen Atemgasströmung in den Exspirationskanal (38) sowie eine Einlass-Anschlussformation (30a) aufweist, die zum Anschluss an eine zum Patienten führende Exspirations-Atemgasleitung (36a) ausgebildet ist, und welcher an seinem anderen Ende einen Exspirationsauslass (40) zum Abblasen von exspiratorischem Atemgas aufweist, wobei der Exspirationskanal (38) ein Exspirationsventil (42) aufweist, welches durch eine exspiratorische Atemgasströmung in Exspirationsrichtung (E) vom Exspirationseinlass (22) zum Exspirationsauslass (40) in eine die exspiratorische Atemgasströmung durchlassende Durchlassstellung bewegbar ist,
- einen Inspirationskanal (52), welcher an einem Ende einen Inspirationseinlass (54) zum Einleiten einer inspiratorischen Atemgasströmung in den Inspirationskanal (52) sowie eine Einlass-Verbindungsformation (58) aufweist, die zur Verbindung mit einer inspiratorisches Atemgas liefernden Atemgasquelle ausgebildet ist, und welcher an seinem anderen Ende einen Inspirationsauslass (24) zum Auslassen von inspiratorischem Atemgas sowie eine Auslass-Verbindungsformation (30b) aufweist, die zur Verbindung mit einer zum Patienten führenden Inspirations-Atemgasleitung (36b) ausgebildet ist,
- einen Steuerkanal (68), welcher an einem Abzweigort (66) von dem Inspirationskanal (52) abzweigt und derart zu dem Exspirationsventil (42) führt, dass das Exspirationsventil (42) durch inspiratorisches Atemgas in eine die exspiratorische Atemgasströmung sperrende Sperrstellung belastbar ist,
**dadurch gekennzeichnet, dass** im Inspirationskanal (52) ein Einwegventil (60, 60') angeordnet ist, welches eine inspiratorische Atemgasströmung in Inspirationsrichtung (I) vom Inspirationseinlass (54) zum Inspirationsauslass (24) zulässt und eine Atemgasströmung in entgegengesetzter Richtung unterbindet.

2. Exspirationsventil-Baugruppe (16) nach Anspruch 1,
**dadurch gekennzeichnet, dass** der Abzweigort (66) in Inspirationsrichtung (I) stromaufwärts des Einwegventils (60, 60') gelegen ist.

3. Exspirationsventil-Baugruppe (16) nach Anspruch 1 oder 2,
**dadurch gekennzeichnet, dass** ein dem Inspirationseinlass (54) näher als dem Inspirationsauslass (24) gelegener Inspirations-Einlassabschnitt (56) des Inspirationskanals (52) längs einer Inspirations-Einlassachse (IEA) verläuft, wobei eine Ventilbewegungsbahn (VBB), längs welcher ein Ventilkörper (46) des Exspirationsventils (42) von einem Ventilsitz (44) des Exspirationsventils (42) aus seiner Sperrstellung abhebbar und an den Ventilsitz (44) annäherbar ist, relativ zur Inspirations-Einlassachse (IEA) um einen Anstellwinkel (α) im Bereich von 10° bis 45°, vorzugsweise im Bereich von 15° bis 35°, geneigt ist.

4. Exspirationsventil-Baugruppe (16) nach Anspruch 3,
**dadurch gekennzeichnet, dass** ein dem Exspirationseinlass (22) näher als dem Exspirationsauslass (40) gelegener Exspirations-Einlassabschnitt (26) des Exspirationskanals längs einer Exspirations-Einlassachse (EEA) verläuft, wobei die Ventilbewegungsbahn (VBB) um eine Parallele (P) zur Exspirations-Einlassachse (EAA) bezüglich der Inspirations-Einlassachse (IAA) geneigt ist.

5. Exspirationsventil-Baugruppe (16) nach Anspruch 3 oder 4,
**dadurch gekennzeichnet, dass** der Ventilsitz (44) einen dem Inspirations-Einlassabschnitt (56) zugeneigten, angenäherten Näherungsabschnitt (44a) und einen vom Inspirations-Einlassabschnitt (56) weg geneigten, weiter entfernt gelegenen Distanzabschnitt (44b) aufweist, wobei der Steuerkanal (68) näher beim Näherungsabschnitt (44a) als beim Distanzabschnitt (44b) vom Abzweigort (66) zum Exspirationsventil (42) verläuft.

6. Exspirationsventil-Baugruppe (16) nach Anspruch 4 oder nach Anspruch 5, rückbezogen auf Anspruch 4,
**dadurch gekennzeichnet, dass** in einem Bereich zwischen dem Inspirations-Einlassabschnitt (56) und dem Exspirations-Einlassabschnitt (30a) oder/und in einem Bereich zwischen dem Inspirations-Einlassabschnitt (56) und einem dem Inspirationsauslass (24) näher als dem Inspirationseinlass (54) gelegenen Inspirations-Auslassabschnitt (28) wenigstens eine Durchlassöffnung (72a, 72b) ausgebildet ist, welche eine den Inspirationskanal (52) oder/und den Exspirationskanal (38) begrenzende Kanalwand (52a, 38c) durchsetzt.

7. Exspirationsventil-Baugruppe (16) nach Anspruch 6,
**dadurch gekennzeichnet, dass** die wenigstens eine Durchlassöffnung (72a, 72b) in Exspirationsrichtung (E) stromabwärts einer Bezugsebene (BE) gelegen ist, welche zur Exspirations-Einlassachse (EEA) orthogonal orientiert ist und eine vom Ventilsitz (44) eingefasste Öffnungsfläche in gleiche Flächenteile unterteilt.

8. Exspirationsventil-Baugruppe (16) nach Anspruch 6 oder 7,
**dadurch gekennzeichnet, dass** die wenigstens eine Durchlassöffnung (72a, 72b) längs einer Durchlassachse (DA) verläuft, welche parallel ist zur Exspirations-Einlassachse (EEA) oder/und zu einer Inspirations-Auslassachse (IAA), längs welcher sich der Inspirations-Auslassabschnitt (28) erstreckt.

9. Exspirationsventil-Baugruppe (16) nach einem der vorhergehenden Ansprüche, unter Einbeziehung der Ansprüche 4 und 6,
**dadurch gekennzeichnet, dass** der Exspirations-Einlassabschnitt (26) und der Inspirations-Auslassabschnitt (28) in einem gemeinsamen Multi-Lumen-Endabschnitt (20) ausgebildet sind.

10. Exspirationsventil-Baugruppe (16) nach einem der vorhergehenden Ansprüche, unter Einbeziehung der Ansprüche 4 und 6,
**dadurch gekennzeichnet, dass** der Inspirations-Einlassabschnitt (56), der Exspirations-Einlassabschnitt (26) und der Inspirations-Auslassabschnitt (28) durch ein einstückiges Kanalbauteil (18) realisiert sind.

11. Atemgas-Leitungsbaugruppe (10), umfassend eine Exspirationsventil-Baugruppe (16) nach einem der vorhergehenden Ansprüche, eine Exspirations-Atemgasleitung (36a), eine Inspirations-Atemgasleitung (36b) sowie einen Durchflusssensor (108),
wobei die Exspirations-Atemgasleitung (36a) an ihrem distalen Längsende eine distale exspiratorische Kopplungsformation (34 aufweist, welche zur Herstellung einer eine exspiratorische Atemgasströmung leitenden Verbindung mit der Einlass-Anschlussformation (26) des Exspirationskanals (58) der Exspirationsventil-Baugruppe (16) ausgebildet ist,
wobei die Exspirations-Atemgasleitung (36a) an ihrem proximalen Längsende eine proximale exspiratorische Kopplungsformation (94a) aufweist, welche zur Herstellung einer eine exspiratorische Atemgasströmung leitenden Verbindung mit einer exspiratorischen Ausgabe-Anschlussformation (109) am distalen Längsende des Durchflusssensors (108) ausgebildet ist,
wobei die Inspirations-Atemgasleitung (36b) an ihrem distalen Längsende eine distale inspiratorische Kopplungsformation (34) aufweist, welche zur Herstellung einer eine inspiratorische Atemgasströmung leitenden Verbindung mit der Auslass-Verbindungsformation (28) des Inspirationskanals (52) der Exspirationsventil-Baugruppe (16) ausgebildet ist,
wobei die Inspirations-Atemgasleitung (36b) an ihrem proximalen Längsende eine proximale inspiratorische Kopplungsformation (94b) aufweist, welche zur Herstellung einer eine inspiratorische Atemgasströmung leitenden Verbindung mit einer inspiratorischen Eingabe-Anschlussformation (109) am distalen Längsende des Durchflusssensors (108) ausgebildet ist,
wobei in wenigstens einer Atemgasleitung (36a, 36b) aus Exspirations- und Inspirations-Atemgasleitung (36a, 36b) wenigstens eine Signalleitung (78, 80) aufgenommen ist, welche dazu ausgebildet ist, eine Erfassungsinformation des Durchflusssensors (108) vom proximalen zum distalen Längsende der wenigstens einen Atemgasleitung (36a, 36b) zu übertragen.

12. Atemgas-Leitungsbaugruppe (10) nach Anspruch 11, unter Einbeziehung des Anspruchs 6,
**dadurch gekennzeichnet, dass** wenigstens eine der wenigstens einen Signalleitung (78, 80) wenigstens eine der wenigstens einen Durchlassöffnung (72a, 72b) durchsetzt oder in Aufnahmeeingriff einer Aufnahmeformation (76) zur Aufnahme des distalen Endes der wenigstens einen Signalleitung (78, 80) endet.

13. Atemgas-Leitungsbaugruppe (10) nach Anspruch 11 oder 12,
**dadurch gekennzeichnet, dass** die Exspirations-Atemgasleitung (36a) und die Inspirations-Atemgasleitung (36b) an einem gemeinsamen Multi-Lumen-Leitungsbauteil (36) ausgebildet sind.

14. Atemgas-Leitungsbaugruppe (10) nach Anspruch 13,
**dadurch gekennzeichnet, dass** im Exspirationslumen (36a) und im Inspirationslumen (36b) je eine Signalleitung (78, 80) aufgenommen ist.

15. Atemgas-Leitungsbaugruppe (10) nach einem der Ansprüche 13 bis 14, **dadurch gekennzeichnet, dass** Atemgas-Leitungsbaugruppe (10) ein proximales Kopplungsbauteil (90) aufweist, mit welchem das Multi-Lumen-Leitungsbauteil (36) verbunden ist, wobei das Kopplungsbauteil (90) an seinem distalen Längsende eine Exspirations-Verbindungsformation (90a) zur Herstellung einer eine exspiratorische Atemgasströmung leitenden Verbindung mit dem Exspirationslumen (36a) und eine Inspirations-Verbindungsformation (90b) zur Herstellung einer eine inspiratorische Atemgasströmung leitenden Verbindung mit dem Inspirationslumen (36b) aufweist, und wobei das das Kopplungsbauteil (90) an seinem proximalen Längsende eine Kopplungsformation (94) aufweist, welche sowohl die proximale exspiratorische Kopplungsformation (94a) als auch die proximale inspiratorische Kopplungsformation (94b) ist.

## Claims

1. Expiration valve assembly (16) for a respiratory device for artificial ventilation of a patient, comprising
- An expiration duct (38) which at one end exhibits an expiration inlet (22) for introducing an expiratory breathing gas flow into the expiration duct (38) and an inlet linkage formation (30a) which is configured for linking to an expiration breathing gas line (36a) leading to the patient, and which at its other end exhibits an expiration outlet (40) for discharging expiratory breathing gas, where the expiration duct (38) exhibits an expiration valve (42) which through an expiratory breathing gas flow in the expiration direction (E) from the expiration inlet (22) to the expiration outlet (40) is moveable into a feed-through position which lets the expiratory breathing gas flow through,
- An inspiration duct (52) which exhibits at one end an inspiration inlet (54) for introducing an inspiratory breathing gas flow into the inspiration duct (52) and an inlet connector formation (58) which is configured for connecting with a breathing gas source supplying inspiratory breathing gas, and which at its other end exhibits an inspiration outlet (24) for letting out inspiratory breathing gas and an outlet connector formation (30b) which is configured for connecting with an inspiration breathing gas line (36b) leading to the patient,
- A control duct (68) which branches off from the inspiration duct (52) at a branching point (66) and leads to the expiration valve (42) in such a way that the expiration valve (42) can be strained by inspiratory breathing gas into a blocking position which blocks the expiratory breathing gas flow,
**Characterized in that** in the inspiration duct (52) there is arranged a one-way valve (60, 60') which allows inspiratory breathing gas flow in the inspiration direction (I) from the inspiration inlet (54) to the inspiration outlet (24) and prevents breathing gas flow in the opposite direction.

2. Expiration valve assembly (16) according to Claim 1,
**Characterized in that** the branching point (66) lies upstream of the one-way valve (60, 60') in the inspiration direction (I).

3. Expiration valve assembly (16) according to Claim 1 or 2,
**Characterized in that** an inspiration inlet section (56) of the inspiration duct (52) which lies nearer to the inspiration inlet (54) than to the inspiration outlet (24) proceeds along an inspiration inlet axis (IEA), where a valve movement path (VBB) along which a valve body (46) of the expiration valve (42) can be raised from a valve seat (44) of the expiration valve (42) from its blocking position and made to approach the valve seat (44), is tilted relative to the inspiration inlet axis (IEA) by a setting angle (α) in the range from 10° to 45°, preferably in the range from 15° to 35°.

4. Expiration valve assembly (16) according to Claim 3,
**Characterized in that** an expiration inlet section (26) of the expiration duct which lies nearer to the expiration inlet (22) than to the expiration outlet (40) proceeds along an expiration inlet axis (EEA), where the valve movement path (VBB) is tilted about a parallel (P) to the expiration inlet axis (EAA) with respect to the inspiration inlet axis (IAA).

5. Expiration valve assembly (16) according to Claim 3 or 4,
**Characterized in that** the valve seat (44) exhibits a proximity section (44a) approaching and tilted towards the inspiration inlet section (56) and a distanced section (44b) lying further away and tilted away from the inspiration inlet section (56), where the control duct (68) runs nearer to the proximity section (44a) than to the distanced section (44b) from the branching point (66) to the expiration valve (42).

6. Expiration valve assembly (16) according to Claim 4 or according to Claim 5, by reference to Claim 4,
**Characterized in that** in a region between the inspiration inlet section (56) and the expiration inlet section (30a) and/or in a region between the inspiration inlet section (56) and an inspiration outlet section (28) lying nearer to the inspiration outlet (24) than to the inspiration inlet (54) there is configured at least one feed-through aperture (72a, 72b) which penetrates through a duct wall (52a, 38c) which bounds the inspiration duct (52) and/or the expiration duct (38).

7. Expiration valve assembly (16) according to Claim 6,
**Characterized in that** the at least one feed-through aperture (72a, 72b) is located in the expiration direction (E) downstream of a reference plane (BE) which is oriented orthogonally to the expiration inlet axis (EEA) and subdivides an aperture surface bordered by the valve seat (44) into equal area parts.

8. Expiration valve assembly (16) according to Claim 6 or 7,
**Characterized in that** the at least one feed-through aperture (72a, 72b) runs along a feed-through axis (DA) which is parallel to the expiration inlet axis (EEA) and/or to an inspiration outlet axis (IAA) along which the inspiration outlet section (28) extends.

9. Expiration valve assembly (16) according to one of the preceding Claims, in consideration of Claims 4 and 6,
**Characterized in that** the expiration inlet section (26) and the inspiration outlet section (28) are configured in a common multi-lumen end section (20).

10. Expiration valve assembly (16) according to one of the preceding Claims, in consideration of Claims 4 and 6,
**Characterized in that** the inspiration inlet section (56), the expiration inlet section (26), and the inspiration outlet section (28) are realized through an integral duct component (18).

11. Breathing gas line assembly (10), comprising an expiration valve assembly (16) according to one of the preceding Claims, an expiration breathing gas line (36a), an inspiration breathing gas line (36b), and a flow sensor (108),
Where the expiration breathing gas line (36a) exhibits at its distal longitudinal end a distal expiratory coupling formation (34) which is configured to establish a connection which conducts an expiratory breathing gas flow with the inlet linkage formation (26) of the expiration duct (58) of the expiration valve assembly (16),
Where the expiration breathing gas line (36a) exhibits at its proximal longitudinal end a proximal expiratory coupling formation (94a) which is configured to establish a connection which conducts an expiratory breathing gas flow with an expiratory output linkage formation (109) at the distal longitudinal end of the flow sensor (108),
Where the inspiration breathing gas line (36b) exhibits at its distal longitudinal end a distal inspiratory coupling formation (34) which is configured to establish a connection which conducts an inspiratory breathing gas flow with the outlet connector formation (28) of the inspiration duct (52) of the expiration valve assembly (16),
Where the inspiration breathing gas line (36b) exhibits at its proximal longitudinal end a proximal inspiratory coupling formation (94b) which is configured to establish a connection which conducts an inspiratory breathing gas flow with an inspiratory input linkage formation (109) at the distal longitudinal end of the flow sensor (108),
Where in at least one breathing gas line (36a, 36b) out of the expiration and inspiration breathing gas line (36a, 36b) there is accommodated at least one signaling line (78, 80) which is configured to transmit information acquired by the flow sensor (108) from the proximal to the distal longitudinal end of the at least one breathing gas line (36a, 36b).

12. Breathing gas line assembly (10) according to Claim 11, in consideration of Claim 6,
**Characterized in that** at least one of the at least one signaling line (78, 80) penetrates through at least one of the at least one feed-through aperture (72a, 72b) or ends in an accommodating engagement of an accommodating formation (76) for accommodating the distal end of the at least one signaling line (78, 80).

13. Breathing gas line assembly (10) according to Claim 11 or 12,
**Characterized in that** the expiration breathing gas line (36a) and the inspiration breathing gas line (36b) are configured at a common multi-lumen line component (36).

14. Breathing gas line assembly (10) according to Claim 13,
**Characterized in that** in the expiration lumen (36a) and in the inspiration lumen (36b) there is a signaling line (78, 80) accommodated in each.

15. Breathing gas line assembly (10) according to one of the Claims 13 to 14, **Characterized in that** the breathing gas line assembly (10) exhibits a proximal coupling component (90) with which the multi-lumen line component (36) is connected, where the coupling component (90) exhibits at its distal longitudinal end an expiration connector formation (90a) for establishing a connection which conducts an expiratory breathing gas flow with the expiration lumen (36a) and an inspiration connector formation (90b) for establishing a connection which conducts an inspiratory breathing gas flow with the inspiration lumen (36b), and where the coupling component (90) exhibits at its proximal longitudinal end a coupling formation (94) which is both the proximal expiratory coupling formation (94a) and the proximal inspiratory coupling formation (94b).

## Revendications

1. Ensemble de soupape d'expiration (16) pour un dispositif de ventilation destiné à la ventilation artificielle d'un patient, comprenant
- un canal d'expiration (38) qui présente à une extrémité une entrée d'expiration (22) pour introduire un écoulement de gaz respiratoire expiratoire dans le canal d'expiration (38) ainsi qu'une formation de raccordement d'entrée (30a) qui est conçue pour le raccordement à une conduite de gaz respiratoire expiratoire (36a) menant au patient, et qui présente à son autre extrémité une sortie d'expiration (40) pour évacuer le gaz respiratoire expiratoire, le canal d'expiration (38) présentant une soupape d'expiration (42) qui peut être déplacée par un écoulement de gaz respiratoire expiratoire dans le sens de l'expiration (E) de l'entrée d'expiration (22) vers la sortie d'expiration (40) dans une position de passage laissant passer l'écoulement de gaz respiratoire expiratoire,
- un canal d'inspiration (52) qui présente à une extrémité une entrée d'inspiration (54) pour l'introduction d'un écoulement de gaz respiratoire inspiratoire dans le canal d'inspiration (52) ainsi qu'une formation de liaison d'entrée (58) qui est conçue pour la liaison avec une source de gaz respiratoire fournissant du gaz respiratoire inspiratoire, et qui présente à son autre extrémité une sortie d'inspiration (24) pour l'évacuation du gaz respiratoire inspiratoire ainsi qu'une formation de liaison de sortie (30b) qui est conçue pour la liaison avec une conduite de gaz respiratoire inspiratoire (36b) menant au patient,
- un canal de commande (68) qui part du canal d'inspiration (52) à un emplacement de dérivation (66) et qui mène à la soupape d'expiration (42) de telle sorte que la soupape d'expiration (42) peut être sollicitée par du gaz respiratoire inspiratoire dans une position de blocage bloquant l'écoulement de gaz respiratoire expiratoire,
**caractérisé en ce qu'**une soupape unidirectionnelle (60, 60') est disposée dans le canal d'inspiration (52), laquelle autorise un écoulement de gaz respiratoire inspiratoire dans le sens de l'inspiration (I) de l'entrée d'inspiration (54) vers la sortie d'inspiration (24) et empêche un écoulement de gaz respiratoire dans le sens opposé..

2. Ensemble de soupape d'expiration (16) selon la revendication 1, **caractérisé en ce que** l'emplacement de dérivation (66) est situé en amont de la soupape unidirectionnelle (60, 60') dans le sens de l'inspiration (I).

3. Ensemble de soupape d'expiration (16) selon la revendication 1 ou 2, **caractérisé en ce qu'**une partie d'entrée d'inspiration (56) du canal d'inspiration (52) située plus près de l'entrée d'inspiration (54) que de la sortie d'inspiration (24) s'étend le long d'un axe d'entrée d'inspiration (IEA), une trajectoire de déplacement de soupape (VBB), le long de laquelle un corps de soupape (46) de la soupape d'expiration (42) peut être soulevé d'un siège de soupape (44) de la soupape d'expiration (42) à partir de sa position de blocage et peut être rapproché du siège de soupape (44), est incliné par rapport à l'axe d'entrée d'inspiration (IEA) d'un angle d'inclinaison (α) dans la plage de 10° à 45°, de préférence dans la plage de 15° à 35°.

4. Ensemble de soupape d'expiration (16) selon la revendication 3, **caractérisé en ce qu'**une partie d'entrée d'expiration (26) du canal d'expiration située plus près de l'entrée d'expiration (22) que de la sortie d'expiration (40) s'étend le long d'un axe d'entrée d'expiration (EEA), la trajectoire de déplacement de soupape (VBB) étant inclinée d'une parallèle (P) à l'axe d'entrée d'expiration (EAA) par rapport à l'axe d'entrée d'inspiration (IAA).

5. Ensemble de soupape d'expiration (16) selon la revendication 3 ou 4, **caractérisé en ce que** le siège de soupape (44) comprend une partie de proximité (44a) proche de la partie d'entrée d'inspiration (56) et une partie d'espacement (44b) plus éloignée, inclinée à l'écart de la partie d'entrée d'inspiration (56), le canal de commande (68) s'étendant plus près de la partie de proximité (44a) que de la partie d'espacement (44b) depuis l'emplacement de dérivation (66) vers la soupape d'expiration (42).

6. Ensemble de soupape d'expiration (16) selon la revendication 4 ou selon la revendication 5, en référence à la revendication 4,
**caractérisé en ce que** dans une zone entre la section d'entrée d'inspiration (56) et la section d'entrée d'expiration (30a) ou/et dans une zone entre la section d'entrée d'inspiration (56) et une section de sortie d'inspiration (28) située plus près de la sortie d'inspiration (24) que de l'entrée d'inspiration (54), au moins une ouverture de passage (72a, 72b) est formée, laquelle traverse une paroi de canal (52a, 38c) délimitant le canal d'inspiration (52) ou/et le canal d'expiration (38).

7. Ensemble de soupape d'expiration (16) selon la revendication 6,
**caractérisé en ce que** ladite au moins une ouverture de passage (72a, 72b) est située en aval, dans la direction d'expiration (E), d'un plan de référence (BE) qui est orienté orthogonalement à l'axe d'entrée d'expiration (EEA) et qui divise une surface d'ouverture bordée par le siège de soupape (44) en parties de surface égales.

8. Ensemble de soupape d'expiration (16) selon la revendication 6 ou 7,
**caractérisé en ce que** ladite au moins une ouverture de passage (72a, 72b) s'étend le long d'un axe de passage (DA) qui est parallèle à l'axe d'entrée d'expiration (EEA) ou/et à un axe de sortie d'inspiration (IAA) le long duquel s'étend la section de sortie d'inspiration (28).

9. Ensemble de soupape d'expiration (16) selon l'une quelconque des revendications précédentes, en incluant les revendications 4 et 6,
**caractérisé en ce que** la section d'entrée d'expiration (26) et la section de sortie d'inspiration (28) sont formées dans une section d'extrémité multi-lumière commune (20).

10. Ensemble de soupape d'expiration (16) selon l'une quelconque des revendications précédentes, incluant les revendications 4 et 6,
**caractérisé en ce que** la section d'entrée d'inspiration (56), la section d'entrée d'expiration (26) et la section de sortie d'inspiration (28) sont réalisées par un composant de conduit monobloc (18).

11. Ensemble de conduite de gaz respiratoire (10) comprenant un ensemble de soupape d'expiration (16) selon l'une quelconque des revendications précédentes, un conduit de gaz respiratoire d'expiration (36a), un conduit de gaz respiratoire inspiratoire (36b) et un capteur de débit (108),
dans lequel le conduit de gaz respiratoire expiré (36a) présente, à son extrémité longitudinale distale, une formation de couplage expiratoire distale (34) qui est conçue pour établir une liaison conduisant un écoulement de gaz respiratoire expiratoire avec la formation de raccordement d'entrée (26) du canal d'expiration (58) de l'ensemble de soupape d'expiration (16),
dans lequel le conduit de gaz respiratoire expiratoire (36a) comprend, à son extrémité longitudinale proximale, une formation de couplage expiratoire proximale (94a) qui est configurée pour établir une connexion conduisant un écoulement de gaz respiratoire expiratoire avec une formation de connexion de sortie expiratoire (109) à l'extrémité longitudinale distale du capteur de débit (108),
dans lequel le conduit de gaz respiratoire inspiratoire (36b) présente, à son extrémité longitudinale distale, une formation de couplage inspiratoire distale (34) qui est conçue pour établir une liaison conduisant un écoulement de gaz respiratoire inspiratoire avec la formation de liaison de sortie (28) du canal d'inspiration (52) de l'ensemble de soupape d'expiration (16),
dans lequel le conduit de gaz respiratoire inspiratoire (36b) présente à son extrémité longitudinale proximale une formation de couplage inspiratoire proximale (94b) qui est conçue pour établir une liaison conduisant un écoulement de gaz respiratoire inspiratoire avec une formation de raccordement d'entrée inspiratoire (109) à l'extrémité longitudinale distale du capteur de débit (108),
au moins une ligne de signal (78, 80) étant logée dans au moins une conduite de gaz respiratoire (36a, 36b) parmi la conduite de gaz respiratoire expiratoire et inspiratoire (36a, 36b), laquelle est conçue pour transmettre une information de détection du capteur de débit (108) de l'extrémité longitudinale proximale à l'extrémité longitudinale distale de la au moins une conduite de gaz respiratoire (36a, 36b).

12. Ensemble de conduit de gaz respiratoire (10) selon la revendication 11, en incluant la revendication 6,
**caractérisé en ce qu'**au moins une de ladite au moins une ligne de signal (78, 80) traverse au moins une de ladite au moins une ouverture de passage (72a, 72b) ou se termine en prise de réception d'une formation de réception (76) pour recevoir l'extrémité distale de ladite au moins une ligne de signal (78, 80).

13. Ensemble de conduit de gaz respiratoire (10) selon la revendication 11 ou 12, **caractérisé en ce que** le conduit de gaz respiratoire expiratoire (36a) et le conduit de gaz respiratoire inspiratoire (36b) sont formés sur un composant de conduit multi-lumière commun (36).

14. Ensemble de conduits de gaz respiratoire (10) selon la revendication 13, **caractérisé en ce qu'**une ligne de signalisation (78, 80) est logée dans la lumière d'expiration (36a) et dans la lumière d'inspiration (36b).

15. Ensemble de conduits de gaz respiratoire (10) selon l'une des revendications 13 à 14,
**caractérisé en ce que** l'ensemble de conduit de gaz respiratoire (10) comprend un élément de couplage proximal (90) auquel l'élément de conduit multi-lumière (36) est connecté, l'élément de couplage (90) ayant à son extrémité longitudinale distale une formation de connexion d'expiration (90a) pour établir une connexion conduisant un écoulement de gaz respiratoire expiratoire avec la lumière d'expiration (36a) et une formation de connexion d'inspiration (90b) pour établir une connexion conduisant un écoulement de gaz respiratoire inspiratoire avec la lumière d'inspiration (36b), et dans lequel l'élément d'accouplement (90) comprend, à son extrémité longitudinale proximale, une formation d'accouplement (94) qui est à la fois la formation d'accouplement expiratoire proximale (94a) et la formation d'accouplement inspiratoire proximale (94b).
